# EUROPEAN PATENT SPECIFICATION

(11) **EP 3 709 023 B1**
(45) Date of publication and mention of the grant of the patent: **16.03.2022**
(21) Application number: 20163421.9
(22) Date of filing: 16.03.2020
(51) Int. Cl.: G01N 33/68, G01N 30/00, B01D 15/00

(54) **DEVICES AND METHODS FOR ISOLATING MATTERS OF INTEREST**
VORRICHTUNGEN UND VERFAHREN ZUR ISOLIERUNG VON MATERIALIEN VON INTERESSE
DISPOSITIFS ET PROCÉDÉS PERMETTANT D'ISOLER DES MATIÈRES D'INTÉRÊT

(30) Priority: 15.03.2019 US 201962818756 P
(43) Date of publication of application: 16.09.2020
(73) Proprietor: Yang, Kuen-Der, Taipei City 103 (TW)
(72) Inventor: Yang, Kuen-Der, Taipei City 103 (TW)
(74) Representative: Lang, Christian

(56) References cited:
- WO-A1-2007/127848
- WO-A1-2012/115885
- WO-A1-2015/113699
- WO-A1-2018/018707
- US-A- 5 716 526

## Description

### BACKGROUND OF THE INVENTION

### 1. FIELD OF THE INVENTION

The present disclosure in general relates to the field of separating and isolating matters of interest (MOTs) from a biological sample.

### 2. DESCRIPTION OF RELATED ART

Cells, extracellular vesicles (EVs) including exosomes (Exo) and microvesicles (MV), and soluble molecules are present in various sources of tissue fluids, such as urine, cerebrospinal fluid, pleural fluids, ascites, amniotic fluid, bronchoalveolar lavage and saliva. Since the proteins and/or nucleic acid level of cells, MV, Exo and/or soluble molecules reflect the status of human physiology and pathology of tissues, thus cells, Exo and/or soluble molecules are suitable means for prediction, diagnosis, and prognosis of diseases, in which the proteins and/or nucleic acid levels of cells, MV, Exo and/or soluble molecules may be determined and compared with those of a normal healthy subject.

As cells, MV, Exo and/or soluble molecules may derive from various types of cells (e.g., endothelial cells, epithelial cells, leukocytes, and etc), whether sick or normal; and may be released from remote tissues (e.g., brain, heart, pancreas, bone marrow, lungs, kidney, and reproductive tissues including fetal, maternal and placental tissues), thus there exist in the related art a need of a means for isolating cells, MV, Exo and/or soluble molecules from a biological sample of a subject, so that the isolated cells, MV, Exo and/or soluble molecules may be subjected to designated bioanalysis *in situ* or *ex situ* of a biomarker on the isolated cells, MV, Exo and/or soluble molecules, therefore may provide a diagnosis or prognosis on a disease and/or condition related to the analyzed biomarker of the subject. Preferably, based on the analyzed biomarker, a proper treatment may be administered to the subject to alleviate and/or ameliorate symptoms associated with the disease and/or condition.

Document US 5 716 526 A teaches a conventional method and device for separating liposomes or lipid complexes from a fluidic medium by using a composite filter consisting of a ceramic membrane and a ceramic substrate. Document WO 2007/127848 A1 discloses a method of isolating membrane vesicles from a biological fluid sample. Document WO 2012/115885 A1 further relates to a method for detecting one or more biomarker in a biological sample, wherein the biological sample comprises an extracellular microvesicle population being isolated from the biological sample prior to the identifying step. From document WO 2015/113699 A1 a filtering device is known which is configured for removing substances from blood or a blood component and comprising a housing, a first sorbent material coupled with at least a first ligand and a second sorbent material coupled with at least a second ligand, wherein the first ligand is for removing free hemoglobin (fHb) and the second ligand is for removing microvesicles (MV) from the blood or blood component passing through the filtering device. Document WO 2018/018707 A1 further discloses a multi-layered centrifugal filtration device for exosome extraction.

### SUMMARY

The following presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later.

As embodied and broadly described herein, one aspect of the present disclosure is directed to a device for isolating matters of interest (MOIs), namely cells, microvesicles and exosomes, from a biological sample (such as urine sample). The device comprises a cartridge, which comprises three filters configured to retain the three populations of MOIs independently having a size between 1.0-20 µm, 0.20-1.0 µm, or 0.05 -0.20 µm in diameter.

According to the present description, the filter may be a size-exclusion column, an affinity column, an elutriator, or a porous membrane. In one example, the cartridge comprises an affinity column, which is an anion-exchange column. However, according to the present invention, the cartridge comprises three filters, serially connected to each other, in which the first, second and third filters are respectively independently comprising a plurality of pores that are about 0.8-1 µm, 0.20-0.22 µm, and 0.03-0.05 µM in diameter.

According to the present invention, the population having a size between 1.0-20 µm in diameter comprises cells; the population having a size between 0.20-1.0 µm in diameter comprises microvesicles (MVs); and the population having a size between 0.05-0.20 µm in diameter comprises exosomes (Exo).

Alternatively or optionally, the device may further include a first reservoir disposed upstream to the cartridge for housing the sample; and a second reservoir disposed downstream to the cartridge for collecting a filtrate comprising soluble molecules that are smaller than 0.05 µm in diameter.

According to embodiments of the present disclosure, the soluble molecules that are smaller than 0.05 µm in diameter may comprise DNA, RNA, protein, glycan, lipid or a combination thereof.

According to embodiments of the present disclosure, the filters may be made of ceramic, resin, metal, polymer, hollow fiber, or a combination thereof.

According to embodiments of the present disclosure, the ceramic is made of a material selected from the group consisting of zeolite, silica, silicon carbide, alumina, aluminum titanate, spinel, mullite, zirconium phosphate, perovskite, and a combination thereof.

According to embodiments of the present disclosure, the resin is made of a material selected from the group consisting of an organic compound, a synthetic compound and a combination thereof.

According to embodiments of the present disclosure, the metal is selected from the group consisting of stainless steel, nickel, aluminum, silver, gold, cadmium, cobalt, iron, molybdenum, niobium, copper, palladium, platinum, rhodium, ruthenium, tantalum, titanium, tungsten, zirconium, an alloy, and a combination thereof.

According to embodiments of the present disclosure, the polymer is selected from the group consisting of, polycellulose, polyester, poly ether, polypropylene, polyamide, polyimide, polyurethane, polytetrafluoroethylene, polyolefin, polyuria, polyester amide, polyethylene terephthalate, polytetrafluoroethylene, polysiloxane, polysulfone, polyester urethane, polycarbonate, polyvinyl chloride, and a combination thereof.

According to embodiments of the present disclosure, the hollow fiber is a carbon fiber, a glass fiber, a metal fiber, or a combination thereof.

The second aspect of the present disclosure aims to provide a method of isolating cells, microvesicles and exosomes from a urine sample by use of the device described above. The method includes steps of,
(a) allowing the urine sample to pass through the cartridge of the device of claim 1, so as to retain in the three filters the three populations of cells, microvesicles and exosomes independently having a size between 1.0-20 µm, 0.20-1.0 µm, or 0.05-0.20 µm in diameter;
(b) collecting the filtrate eluted from the cartridge, the filtrate comprising soluble molecules that are smaller than 0.05 µm; and
(c) respectively harvesting the cells, microvesicles and exosomes from the filters, and from the filtrate collected in the step (b)

As afore-mentioned, the population having a size between 1.0-20 µm in diameter comprises cells; the population having a size between 0.20-1.0 µm in diameter comprises microvesicles (MVs); the population having a size between 0.05-0.20 µm in diameter comprises exosomes (Exo); and the soluble molecules that are smaller than 0.05 µm in diameter may comprise DNA, RNA, protein, glycan, lipid or a combination thereof.

According to embodiments of the present disclosure, the harvested cells, microvesicles and exosomes obtained in step (c), the filtrate comprising soluble macromolecules obtained in step (b) or the filters from step (a) may be subjected to immunoblotting, chips analysis, fluoroprobing, enzymatic or electrochemical reaction for detecting at least one biomarker thereon or therein.

Examples of such biomarkers include, but are not limited to, soluble fms-like tyrosine kinase 1 (sFlt1), pregnancy associated plasma protein A (PAPPA), brain-derived neurotrophic factor (BNDF), and insulin-like growth factor binding protein 1 (IGFBP1), IGFB2, interleukin-1 (IL-1), IL-2, IL-4, IL-6, IL-8, IL-10, IL-12, interleukin-1 receptor antagonist (IL1-ra), C-X-C Motif Chemokine Ligand 13 (CXCL 13), insulin-like growth factor (IGF), insulin-like growth factor receptor 1 (IGFR1), fibroblast growth factor 1 (FGF-1), FGF-2, leptin, heparin-binding EGF-like growth factor (HB-EGF), vascular endothelial growth factor A (VEGF-A), VEGF-C, VEGF-D, hepatocyte growth factor (HGF), E-cadherin, leucine-rich alpha-2-glycoprotein 1 (LRG1), neutrophil gelatinase-associated lipocalin (NGAL), 8-oxohydroxy deoxyguanosine (8-OHdG), granulocyte colony-stimulating factor (G-CSF), α-synuclein, L1 cell adhesion molecule (L1CAM), S100 calcium-binding protein A8 (S100A8), β4 integrin, mucin 5AC (Muc5AC), amphiregulin (AREG), cell adhesion molecule 1 (CADM1), cochlin, arginase-1 (Arg-1), β-galactosidase, interferon gamma-induced protein 10 (IP-10), monocyte chemoattractant protein 1 (MCP-1), growth-regulated alpha protein (GRO-a), syndecan 1, syndecan 4, monocyte-chemotactic protein-3 (MCP-3), tumor necrosis factor-alpha (TNFα), granulocyte-macrophage colony-stimulating factor (GM-CSF), macrophage colony-stimulating factor (M-CSF), zonulin, neurofilament light (NFL), high mobility group box 1 (HMGB 1), or a nucleic acid.

A further aspect of the present disclosure aims to provide a method of making a diagnosis or prognosis of a disease from a urine sample of a subject with the aid of the present device described above. The urine sample comprises cells, microvesicles, exosomes, and soluble macromolecules, and each of the cells, microvesicles, exosomes, and soluble molecules has a target molecule expressed thereon and/or therein. The method comprises the steps of,
(a) allowing the urine sample to pass through the cartridge of the present device thereby retaining the cells, the microvesicles, and the exosomes by the three filters;
(b) collecting the filtrate downstream the cartridge, which comprises soluble molecules;
(c) adding capture molecules to the harvested soluble molecules in the step (b), and to the cells, the microvesicles, and the exosomes respectively retained by the filters in step (a), wherein each capture molecule is linked with a reporter molecule and exhibits a binding affinity to the target molecule;
(d) determining the level of the reporter molecule bound to the target molecule in the step (c); and
(e) making the diagnosis or prognosis based on the determined level of the reporter molecule in the step (d), wherein, when the determined level of the reporter molecule is different from that of a reference sample obtained from a healthy subject, then the subject has the disease or is at risk of developing the disease.

According to embodiments of the present disclosure, the reporter molecule is selected from the group consisting of, a tag molecule, a radioactive molecule, a fluorescent molecule, a phosphorescent molecule, a chemiluminescent molecule, and an enzyme.

According to embodiments of the present disclosure, in the step (d), the level of the reporter molecule is determined by electrochemical analysis, polymerase chain reaction (PCR), real-time polymerase chain reaction (RT-PCR), flow cytometry assay, enzyme-linked immunosorbent assay (ELISA), chips array, beads array, western blotting, or kinase assay.

According to embodiments of the present disclosure, the capture molecule is an aptamer, a glycan, a lectin, an antibody, or a combination thereof.

According to embodiments of the present disclosure, the target molecule is any of soluble fms-like tyrosine kinase 1 (sFlt1), pregnancy associated plasma protein A (PAPPA), brain-derived neurotrophic factor (BNDF), and insulin-like growth factor binding protein 1 (IGFBP1), IGFBP2, interleukin-1 (IL-1), IL-2, IL-4, IL-6, IL-8, IL-10, IL-12, interleukin-1 receptor antagonist (IL1-ra), C-X-C Motif Chemokine Ligand 13 (CXCL 13), insulin-like growth factor (IGF), insulin-like growth factor receptor 1 (IGFR1), fibroblast growth factor 1 (FGF-1), FGF-2, leptin, heparin-binding EGF-like growth factor (HB-EGF), vascular endothelial growth factor A (VEGF-A), VEGF-C, VEGF-D, hepatocyte growth factor (HGF), E-cadherin, leucine-rich alpha-2-glycoprotein 1 (LRG1), neutrophil gelatinase-associated lipocalin (NGAL), 8-oxohydroxy deoxyguanosine (8-OHdG), granulocyte colony-stimulating factor (G-CSF), α-synuclein, L1 cell adhesion molecule (L1CAM), S100 calcium-binding protein A8 (S100A8), β4 integrin, mucin 5AC (Muc5AC), amphiregulin (AREG), cell adhesion molecule 1 (CADM1), cochlin, arginase-1 (Arg-1), β-galactosidase, interferon gamma-induced protein 10 (IP-10), monocyte chemoattractant protein 1 (MCP-1), growth-regulated alpha protein (GRO-a), syndecan 1, syndecan 4, monocyte-chemotactic protein-3 (MCP-3), tumor necrosis factor-alpha (TNFα), granulocyte-macrophage colony-stimulating factor (GM-CSF), macrophage colony-stimulating factor (M-CSF), zonulin, neurofilament light (NFL), high mobility group box 1 (HMGB 1), or a nucleic acid.

The nucleic acid may be a DNA or an RNA.

According to embodiments of the present disclosure, the disease may be a cancer, an inflammatory disease, a degenerative disease, an infectious disease, or a reproductive disease.

Examples of the cancer include gastric cancer, lung cancer, bladder cancer, breast cancer, pancreatic cancer, renal cancer, colorectal cancer, cervical cancer, ovarian cancer, brain tumor, prostate cancer, hepatocellular carcinoma, melanoma, esophageal carcinoma, multiple myeloma, and head and neck carcinoma.

Examples of the inflammatory disease include psoriasis, colitis, burn injury, acute kidney injury, traumatic brain injury, skin injury, arthritis and autoimmune diseases.

Examples of the degenerative disease include Parkinson's disease, Alzheimer's disease, dementia, stroke, chronic kidney disease, chronic lung disease, benign prostate hypertrophy and hearing loss.

Examples of the infectious disease include infections caused by bacteria, viruses or fungi.

Examples of the reproductive disease include spontaneous abortion, intrauterine retardation, intrauterine infection, congenital anomaly, premature birth, pre-eclampsia, and pregnant diabetes.

The subject from which the sample of the present methods is preferably obtained is a mammal. According to some working examples of the present description, the subject is a human.

Many of the attendant features and advantages of the present disclosure will becomes better understood with reference to the following detailed description considered in connection with the accompanying drawings.

### BRIEF DESCRIPTION OF THE DRAWINGS

The patent or application file contains at least one drawing executed in color. Copies of this patent or patent application publication with color drawing(s) will be provided by the Office upon request and payment of the necessary fee. The present description will be better understood from the following detailed description read in light of the accompanying drawings, where:
FIG 1A is a schematic diagram depicting a device 100 constructed in accordance with one embodiment of the present disclosure;
FIG 1B is a schematic diagram depicting the device 100 of FIG lAin accordance with an optional embodiment of the present disclosure
FIG 2 are photographs illustrating differential expression of E-cadherin;
FIG 3 are photographs depicting isolation of EVs by anion exchange chromatography ,
FIG 4 are photographs illustrating differential expression of (A) neural cell adhesion molecule L1 (LICAM), (B) neuron-specific enolase (NSE), and (C) neutrophil gelatinase-associated lipocalin (NGAL) among cells, MVs, EV between young (Y) and aged (O) subjects ;
FIG 5 are bar graphs depicting differential expression of various vascular mediators and cytokines among cells, MVs, Exo (EV) and the soluble filtrate (Soln) independently isolated by the present device between pregnant women with a normal (N) or premature delivery (P);
FIG 6 are bar graphs depicting differential expression of various biomarkers among MVs, Exo and the soluble filtrate;
FIG 7 are bar graphs depicting differential expression of various biomarkers among MVs, Exo and the soluble filtrate (Soln) between young (AVG-Young) and aged (AVG-old) adult subjects ;
FIG 8 are bar graphs depicting differential expression of various biomarkers among MVs, Exo and the soluble filtrate (Soln) between adults with Parkinson's disease (AVG-PD) and healthy adults with matching age (AVG-C);
FIG 9 are photographs depicting the expression of leucine rich alpha-2-glycoprotein 1 (LRG1) among young subjects (Y1, Y2), aged subjects (O1, O2, O3, and O4) and PD subjects (PD1, PD2);
FIG 10 is a bar graph depicting DNA levels among MVs, Exo and the filtrate (Soln) between young and aged (old) adult subjects;
FIG 11 are bar graphs depicting the level of 8OHdG among MVs, Exo and the soluble filtrate (Soln) between aged subjects with or without PD; and

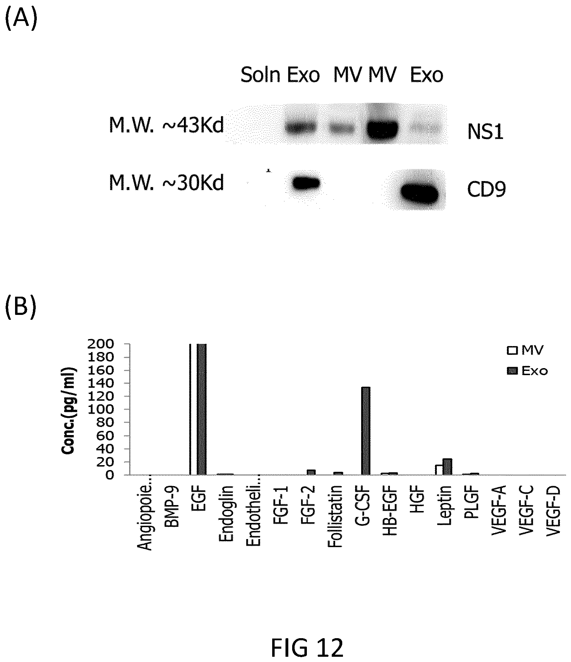

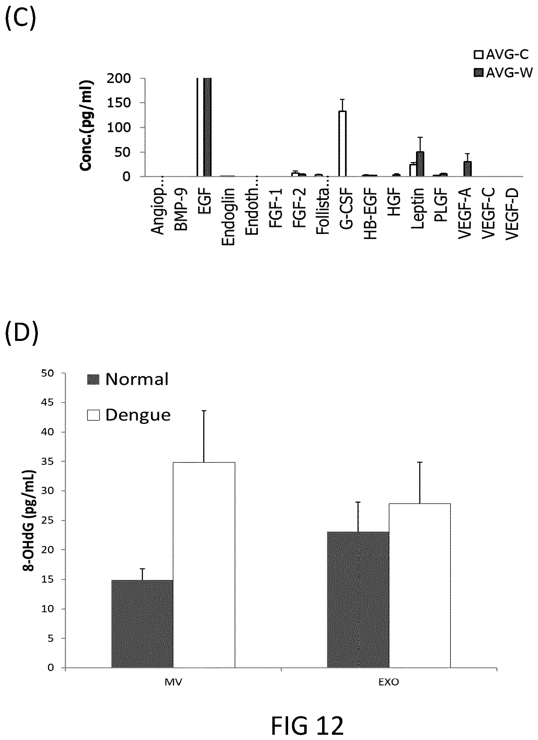

FIG 12 provides data on the levels of dengue virus NS1 antigen, protein biomarkers and DNA among MVs, Exo and the soluble filtrate (Soln) in subjects afflicted with dengue virus infection in accordance with one embodiment of the present disclosure, in which (A) is western blot analysis of NS1 antigen, (B) and (C) are bar graphs respectively depicting differential expression of biomarkers among MV and Exo in dengue subjects that (B) have not and (C) have exhibited clinical warning sign, and (D) is a bar graph depicting the level of 8OHdG among MVs and Exo between healthy subjects and subjects with dengue virus infection.

### DETAILED DESCRIPTION OF THE INVENTION

The detailed description provided below in connection with the appended drawings is intended as a description of the present examples and is not intended to represent the only forms in which the present invention may be constructed or utilized. The description sets forth the functions of the present invention and the sequence of steps for constructing and operating the example. However, the same or equivalent functions and sequences may be accomplished by different examples.

### I. Definition

For convenience, certain terms employed in the specification, examples and appended claims are collected here. Unless otherwise defined herein, scientific and technical terminologies employed in the present disclosure shall have the meanings that are commonly understood and used by one of ordinary skill in the art. Also, unless otherwise required by context, it will be understood that singular terms shall include plural forms of the same and plural terms shall include the singular. Specifically, as used herein and in the claims, the singular forms "a" and "an" include the plural reference unless the context clearly indicates otherwise. Also, as used herein and in the claims, the terms "at least one" and "one or more" have the same meaning and include one, two, three, or more.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about". Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

The term "matters of interest (MOIs)" as used herein refers to cells, vesicles, microvesicles (MVs), and exosomes (Exo) that may be retained by the device of the present disclosure, particularly, those are at least 0.05 µm in diameter, such as 0.06, 0.07, 0.08, 0.09, 0.1, 0.11, 0.12, 0.13, 0.14, 0.15, 0.16, 0.17, 0.18, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.67, 0.66, 0.67, 0.68, 0.069, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, and 1.0 µm in diameter; as well as soluble molecules smaller than 0.05 µm in diameter that are present in the filtrate of the present device (i.e., molecules that pass through the device and end up in the end filtrate), such as soluble molecules like peptides, polypeptides, hormones, growth factors, cytokines and etc.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the expression level of specific target molecule of the present invention, optionally together with other clinical characteristics, to arrive at a diagnosis (that is, the occurrence or nonoccurrence) of a cancer, a degenerative disease, an infectious disease, or aging for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

The term "risk" herein refers to the potential that a result will lead to an undesirable outcome, e.g., occurrence, progression or recurrence of a disease or a condition, for example, a premature delivery of a baby, a degenerative disease, an infectious disease, aging and etc.

The term "administered," "administering" or "administration" are used interchangeably herein to refer a mode of delivery, including, without limitation, intravenously, intraarticularly, intratumorally, intramuscularly, intraperitoneally, intraarterially, intracranially, or subcutaneously administering an agent *(e.g.,* anti-degenerative, anti-infectious, or anti-aging agent) of the present invention.

"Treatment" as used herein includes preventative (e.g., prophylactic), curative or palliative treatment of a disease or condition in a mammal, particularly human; and includes: (1) preventative *(e.g.,* prophylactic), curative or palliative treatment of a disease or condition *(e.g.,* a cancer, degenerative disease, infectious disease, or aging) from occurring in an individual who may be pre-disposed to the disease or condition but has not yet been diagnosed as having it; (2) inhibiting a disease or condition (e.g., by arresting its development); or (3) relieving a disease or condition (e.g., reducing symptoms associated with the disease or condition).

The term "effective amount" as referred to herein designate the quantity of a component which is sufficient to yield a desired response. For therapeutic purposes, the effective amount is also one in which any toxic or detrimental effects of the component are outweighed by the therapeutically beneficial effects. An effective amount of an agent is not required to cure a disease or condition but will provide a treatment for a disease or condition such that the onset of the disease or condition is delayed, hindered or prevented, or the disease or condition symptoms are ameliorated. The effective amount may be divided into one, two, or more doses in a suitable form to be administered at one, two or more times throughout a designated time period. The specific effective or sufficient amount will vary with such factors as the particular condition being treated, the physical condition of the patient *(e.g.,* the patient's body mass, age, or gender), the type of mammal or animal being treated, the duration of the treatment, the nature of concurrent therapy (if any), and the specific formulations employed and the structure of the compounds or its derivatives. Effective amount may be expressed, for example, in cell number, grams, milligrams or micrograms or as milligrams per kilogram of body weight (mg/Kg). Alternatively, the effective amount can be expressed in the concentration of the active component (e.g., anti-degenerative, or anti-infectious agent of the present disclosure), such as cell concentration, molar concentration, mass concentration, volume concentration, molality, mole fraction, mass fraction and mixing ratio. Persons having ordinary skills could calculate the human equivalent dose (HED) for the medicament (such as the present anti-degenerative or anti-infectious agent) based on the doses determined from animal models. For example, one may follow the guidance for industry published by US Food and Drug Administration (FDA) entitled "Estimating the Maximum Safe Starting Dose in Initial Clinical Trials for Therapeutics in Adult Healthy Volunteers" in estimating a maximum safe dosage for use in human subj ects.

The terms "subject" as used in the present disclosure refers to a mammal, for example, a human, a mouse, a rat, a hamster, a guinea pig, a rabbit, a dog, a cat, a cow, a goat, a sheep, a monkey, and a horse. The term "subject" is intended to refer to both the male and female gender unless one gender is specifically indicated. According to preferred embodiments, the subject is a human.

The term "healthy subject" refers to a subject that does not have a disease or condition, *e.g.,* the subject not having a pre-mature delivery symptom, a degenerative disease or an infectious disease, or the subject is not in an aging state *(i.e.,* the subject about or younger than 30 years old). In general, the term "healthy subject" refers to a subject who has not been diagnosed as having a disease or condition, and is not presenting with one or more (e.g., two, three, four or five) symptoms associated with the disease or condition.

### II. Description of The Invention

The invention is defined in the appended claims.

### 1. Device and method for isolation MOIs from a biological sample

The general concept of this specification lies in isolating MOIs (i.e., cells, microvesicles, exosomes, and etc.) from a biological sample, in which the isolation is achieved by size exclusion, or may be otherwise achieved by changes in affinity or ionic strength. Accordingly, a device is constructed by use of multiple filters, which may be a size-exclusion column, an affinity column, an elutriator, or a porous membrane. The thus isolated MOIs may then be subjected to analysis on biomarkers exist therein or thereon, so that diagnosis and/or prognosis of a disease and/or condition of the subject, whom the biological sample derived from may be rendered based on the determined level of the biomarker(s) as compared with that of a control sample, which may be a sample derived from a healthy normal subject.

### 1.1 Isolation of MOIs by size exclusion

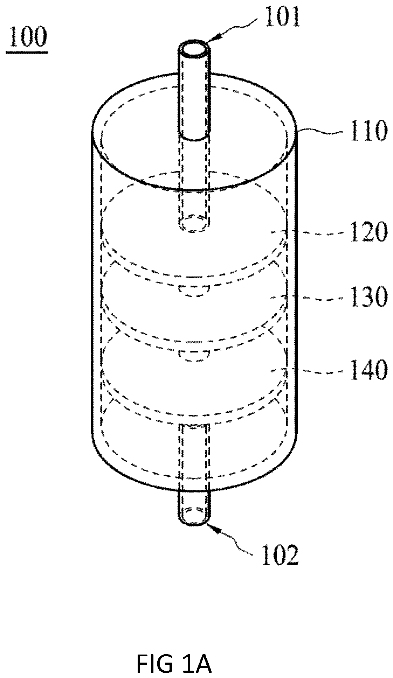

FIG 1A is a schematic diagram depicting a device 100 for isolating MOIs from a biological sample constructed in accordance with Example 1.1 of the present disclosure. The device 100 comprises a cartridge 110, which comprises therein three serially connected filters 120, 130, 140. The three filters 120, 130, 140 are respectively porous membranes and are arranged in the order of decreased pore size. Specifically, from the inlet 101 toward the outlet 102 of the cartridge 110, the pore size of one filter is larger than that of a subsequent filter. In other words, the pore size in the first filter is larger than that of the second filer, which is larger than the third filter. According to the present disclosure, the first filter 120 comprises pores that are about 0.8-1 µm in diameter, the second filter 130 comprises pores that are about 0.20-0.22 µm in diameter, and the third filter 140 comprises pores that are about 0.03-0.05 µm in diameter. By this arrangement of filters, three populations of MOIs independently having a size between 1.0-20 µm, 0.20-1.0 µm, or 0.05-0.20 µm in diameter may be isolated.

During operation, the cartridge 110 is preferably disposed in an up-right position, with the inlet 101 of the cartridge 110 facing upward and the outlet 102 of the cartridge 110 facing downward. An aliquot of a biological sample (e.g., a urine sample) is fed through the inlet 101, and flows downward through the first filter 120, in which MOIs in the biological sample that are 1.0-20 µm in diameter, preferably at least 0.8 µm in diameter (e.g., cells) will be retained by the first filter 120, and the rest of the sample will continue to flow downward and into the second filter 130, where MOIs that are 0.2-1.0 µm in diameter, preferably at least 0.2 µm in diameter (e.g., MVs) are retained by the second filter 130; them the rest of the sample will continue to flow downward into the third filter 140, in which MOIs that are 0.2-0.05 µm in diameter, preferably at least 0.05 µm in diameter (e.g., exosomes) are retained. The rest of the sample containing matters that cannot be retained by any of the three filters 140, 130, 140 will end up in the filtrate, which is collected from the outlet 102 of the cartridge 110. In general, the aliquot of the sample once being fed into the cartridge 110 will flow through the cartridge 110 naturally via the action of gravity. Alternatively or optionally, the sample may also be pushed through with the aid of a force, either manually or automatically, with the aid of a pump, a syringe and the like.

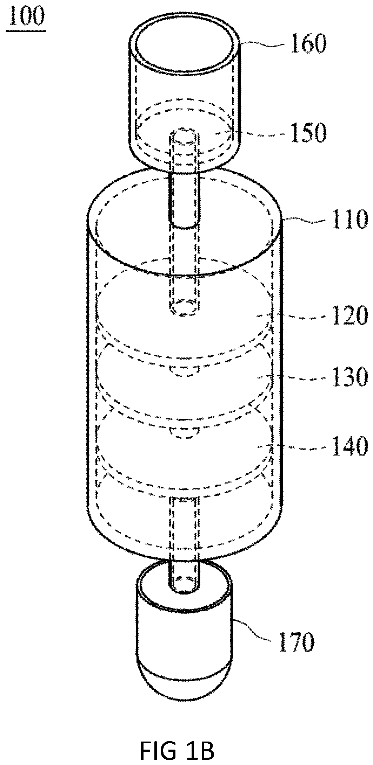

Alternatively or optionally as depicted in FIG 1B, the device 100 may further include a first reservoir 160 disposed upstream to the cartridge 110 for housing the biological sample before and/or during operation. Alternatively or optionally, an additional filter 150 (i.e., a fourth filter 150) may be disposed after the first reservoir 160 and before the cartridge 110, so as to exclude molecules that are greater than 20 µm in diameter in the biological sample from entering the cartridge 110. Alternatively or optionally, a second reservoir 170 may be disposed downstream to the outlet 102 of the cartridge 110 for collecting the filtrate eluted from the device 100.

According to the present disclosure, the MOIs that are retained by the first filter are cells, the MOIs that are retained by the second filter are microvesicles (MVs); and the MOIs retained by the third filter are exosomes (Exos). The filtrate, which is collected from the outlet 102 of the cartridge comprises MOIs (i.e., soluble macromolecules) that are smaller than 0.05 µm in diameter. The MOIs (i.e., soluble molecules) that are smaller than 0.05 µm in diameter comprise DNA, RNA, protein, glycan, lipid or a combination thereof.

MOIs respectively retained by the first, second, and third filters, as well as MOIs that are present in the filtrate may be independently harvested and analyzed for the levels and/or patterns of biomarkers expressed thereon or therein by suitable means specific to the analyzed biomarkers known to the skilled artisan.

### 1.2 Isolation of MOIs by changes in affinity or ionic strength

In an alternative of the present disclosure, not being part of the claimed scope, isolation of MOIs from a biological sample is achieved by use of an affinity column. Accordingly, a device comprises an anion exchange column serving as the filter for isolating MOIs is constructed. An aliquot of the biological sample (e.g., a urine sample) is fed into the device, which comprises an anion exchange column, then MOIs with desired size are eluted by high concentration of salt solution. By this manner, three populations of MOIs independently having a size between 1.0-20 µm, 0.20-1.0 µm or 0.05-0. 20 µm in diameter may be isolated. Specifically, the population having a size between 1.0-20 µm in diameter comprises cells; the population having a size between 0.20-1.0 µm in diameter comprises microvesicles (MVs); the population having a size between 0.05-0. 20 µm in diameter comprises exosomes (Exo).

### 1.3 Filter of the present device

According to embodiments of the present disclosure, the filter suitable for use in the present device and/or method may be made of ceramic, resin, metal, polymer, hollow fiber, or a combination thereof, provided that the material that makes up of the filter is capable of retaining MOIs of the desired size and property.

Examples of the ceramic suitable for constructing the filter of the present device include, but are not limited to, zeolite, silica, silicon carbide, alumina, aluminum titanate, spinel, mullite, zirconium phosphate, perovskite, and a combination thereof.

Examples of the resin suitable for constructing the filter of the present device include, but are not limited to, an organic compound, a synthetic compound and a combination thereof.

Examples of the metal suitable for constructing the filter of the present device include, but are not limited to, stainless steel, nickel, aluminum, silver, gold, cadmium, cobalt, iron, molybdenum, niobium, copper, palladium, platinum, rhodium, ruthenium, tantalum, titanium, tungsten, zirconium, an alloy, and a combination thereof.

Examples of the polymer suitable for constructing the filter of the present device include, but are not limited to, polycellulose, polyester, polyether, polypropylene, polyamide, polyimide, polyurethane, polytetrafluoroethylene, polyolefin, polyuria, polyester amide, polyethylene terephthalate, polytetrafluoroethylene, polysiloxane, polysulfone, polyester urethane, polycarbonate, polyvinyl chloride, and a combination thereof.

Examples of the hollow fiber suitable for constructing the filter of the present device include, but are not limited to, a carbon fiber, a glass fiber, a metal fiber, and a combination thereof.

According to the present disclosure, examples of the biological sample suitable for isolating MOIs therefrom include, but are not limited to, urine, cerebrospinal fluid, pleural fluid, ascites, amniotic fluid, bronchoalveolar lavage, blood, or saliva. Preferably, urine is used for isolating MOIs.

### 2. Use of MOIs retained and/or isolated by the present method and/or device

The device, either comprising one affinity column or, according to the present invention, three serially connected porous membrane filters, may be designed to operate manually or automatically. In manual mode, each filter with the desired MOIs retained therein may be manually dissembled form the cartridge and subjected to subsequent analysis, such as determination of the levels of DNA, biomarkers, and etc., depending on the needs and/or instruction of a user. Alternatively or optionally, the device may be set to operate automatically, in which the device may be coupled to suitable analyzers, so that once the isolation of MOIs is completed, each filter with desired MOIs retained therein is automatically subjected to predesignated assays, such as immunoassays, chromatography analysis, chip array analysis, electrochemical analysis and etc., depending on the needs and/or instruction of a user.

MOIs isolated by the present method and/or device, including MOIs retained by the filter(s), and MOIs that are present in the filtrate may be independently harvested and subjected to analysis for the levels and/or patterns of biomarkers expressed thereon or therein by suitable means specific to the analyzed biomarkers known to the skilled artisan. The determined levels and/or patterns of biomarkers in the MOIs may serve as an indicator for diagnosis and/or prognosis of a pathological condition in a subject.

In one example, the levels and expressed pattern of vascular mediators such as soluble fms-like tyrosine kinase 1 (sFlt1), pregnancy associated plasma protein A (PAPPA), brain-derived neurotrophic factor (BNDF), and insulin-like growth factor binding protein 1 (IGFBP1), IGFBP2, as well as cytokines such as IL-6, IL-8, IL-10, , IL-8, IL-10, C-X-C Motif Chemokine Ligand 13 (CXCL 13), and interleukin-1 receptor antagonist (IL1-ra) are respectively determined in MOIs (including cells, MVs, and exosomes retained by the present device) isolated from urine samples of pregnant women. A relatively higher ratio of sFlt1/IL-10, PAPPA/IL-10 or IL-1ra/IL-10 indicates that a pregnant woman has a higher risk of premature delivery of a baby.

In another example, the levels and/or expressed patterns of biomarkers such as fibroblast growth factor 1 (FGF-1), FGF-2, leptin, heparin-binding EGF-like growth factor (HB-EGF), vascular endothelial growth factor D (VEGF-D) and hepatocyte growth factor (HGF) are respectively determined in MOIs (including cells, MVs, and exosomes retained by the filters, and the filtrate eluted from the cartridge) isolated from urine samples of young or aged subjects. According to one example, aged subjects have high levels of FGF-1 in MVs, VEGF-D in exosomes, and PLGF in the soluble fraction (i.e., the end filtrate of the present method and/or device); while the young subjects have a high level of FGF-2 in MVs.

In a further example, the levels and/or expressed patterns of biomarkers such as FGF-1, HB-EGF and leptin are respectively determined in MOIs (including cells, MVs, and exosomes retained by the filters, and the filtrate eluted from the cartridge) isolated from urine samples of aged subjects with or without Parkinson's disease (PD). According to this example, the isolated exosomes of PD subjects exhibit a higher level of leptin, whereas negligible amount of leptin is found in healthy adults of matching age.

In still further example, the levels of a dengue virus NS1 antigen, as well as the levels and/or expressed patterns of biomarkers such as EGF, G-CSF, VEFG-A, leptin and etc. are respectively determined in MOIs (including cells, MVs, and exosomes retained by the filters, and the filtrate eluted from the cartridge) isolated from urine samples of subjects suffered from dengue virus infection. In this example, significant level of NS1 antigen is found in the MVs isolated from dengue patient. Further, urine MVs and Exos of dengue patient had significant higher level of EGF, while only urine exosomes had expression of G-CSF. Further, dengue subject exhibiting clinical warning sign had higher levels of leptin and VEGF-A, but lower level of G-CSF, as compared with those of dengue patient without warning sign.

### 2.1 Making prognosis of a pathological condition of a subject with the aid of the present device and/or method

Accordingly, the present disclosure also provides a method of making a diagnosis or prognosis of a disease from a biological sample of a subject, in which the biological sample comprises MOIs isolated by the present device and/or method. MOIs thus isolated are cells, microvesicles, exosomes, and soluble macromolecules, and each of the cells, microvesicles, exosomes, and soluble molecules has a target molecule expressed thereon and/or therein. The method comprises steps of,
(a) allowing the biological sample to pass through the cartridge of the present device thereby retaining the cells, the microvesicles, and the exosomes, respectively;
(b) collecting the filtrate of the cartridge, which comprises soluble molecules;
(c) adding capture molecules to the harvested soluble molecules in the step (b), and to the cells, the microvesicles, and the exosomes respectively retained by the filters of the device in the step (a), wherein each capture molecule is linked with a reporter molecule and exhibits a binding affinity to the target molecule;
(d) determining the level of the reporter molecule bound to the target molecule in the step (c); and
(e) making the diagnosis or prognosis based on the determined level of the reporter molecule in the step (d), wherein when the determined level of the reporter molecule is different from that of a reference sample obtained from a healthy subject, then the subject has the disease or is at risk of developing the disease.

Examples of the reporter molecule suitable for use in the present method include a tag molecule, a radioactive molecule, a fluorescent molecule, a phosphorescent molecule, a chemiluminescent molecule, and an enzyme.

According to embodiments of the present disclosure, in the step (d), the level of the reporter molecule is determined by electrochemical analysis, polymerase chain reaction (PCR), real-time polymerase chain reaction (RT-PCR), flow cytometry assay, enzyme-linked immunosorbent assay (ELISA), chips array, beads array, western blotting, or kinase assay.

According to embodiments of the present disclosure, the capture molecule may be an aptamer, a glycan, a lectin, an antibody, or a combination thereof.

Examples of the target molecule on the MOIs include soluble fms-like tyrosine kinase 1 (sFlt1), pregnancy associated plasma protein A (PAPPA), brain-derived neurotrophic factor (BNDF), and insulin-like growth factor binding protein 1 (IGFBP1), IGFBP2, interleukin-1 (IL-1), IL-2, IL-4, IL-6, IL-8, IL-10, IL-12, interleukin-1 receptor antagonist (IL1-ra), C-X-C Motif Chemokine Ligand 13 (CXCL 13), insulin-like growth factor (IGF), insulin-like growth factor receptor 1 (IGFR1), fibroblast growth factor 1 (FGF-1), FGF-2, leptin, heparin-binding EGF-like growth factor (HB-EGF), vascular endothelial growth factor A (VEGF-A), VEGF-C, VEGF-D, hepatocyte growth factor (HGF), E-cadherin, leucine-rich alpha-2-glycoprotein 1 (LRG1), neutrophil gelatinase-associated lipocalin (NGAL), 8-oxohydroxy deoxyguanosine (8-OHdG), granulocyte colony-stimulating factor (G-CSF), α-synuclein, L1 cell adhesion molecule (L1CAM), S100 calcium-binding protein A8 (S100A8), β4 integrin, mucin 5AC (Muc5AC), amphiregulin (AREG), cell adhesion molecule 1 (CADM1), cochlin, arginase-1 (Arg-1), β-galactosidase, interferon gamma-induced protein 10 (IP-10), monocyte chemoattractant protein 1 (MCP-1), growth-regulated alpha protein (GRO-a), syndecan 1, syndecan 4, monocyte-chemotactic protein-3 (MCP-3), tumor necrosis factor-alpha (TNFα), granulocyte-macrophage colony-stimulating factor (GM-CSF), macrophage colony-stimulating factor (M-CSF), zonulin, neurofilament light (NFL), high mobility group box 1 (HMGB1), and a nucleic acid (e.g., DNA and RNA).

According to the present description, an expressed pattern of various specific markers, and/or a ratio of the expressed levels of certain markers detected via suitable methods (e.g., immunoblotting, chips analysis, fluoroprobing, enzymatic or electrochemical reaction) can be used to making a diagnosis and/or prognosis of a disease.

According to embodiments of the present disclosure, the disease that may be diagnosed by the present method include cancer, an inflammatory disease, a degenerative disease, an infectious disease, or a reproductive disease

Examples of the cancer that may be diagnosed by the present method include gastric cancer, lung cancer, bladder cancer, breast cancer, pancreatic cancer, renal cancer, colorectal cancer, cervical cancer, ovarian cancer, brain tumor, prostate cancer, hepatocellular carcinoma, melanoma, esophageal carcinoma, multiple myeloma, and head and neck carcinoma.

Examples of the inflammatory disease that may be diagnosed by the present method include psoriasis, colitis, burn injury, acute kidney injury, traumatic brain injury, skin injury, arthritis and autoimmune diseases.

Examples of the degenerative disease that may be diagnosed by the present method include Parkinson's disease, Alzheimer's disease, dementia, stroke, chronic kidney disease, chronic lung disease, benign prostate hypertrophy and hearing loss.

Examples of the infectious disease that may be diagnosed by the present method include infections caused by bacteria, viruses or fungi.

Examples of the reproductive disease that may be diagnosed by the present method include spontaneous abortion, intrauterine retardation, intrauterine infection, congenital anomaly, premature delivery, pre-eclampsia, and pregnant diabetes.

### 2.2 Method of treating diseases and/or conditions

Also disclosed herein, but not being part of the claimed scope, is a method of treating a premature birth, a degenerative disease or an infectious disease in a subject based on the prognostic or diagnostic result of the method of the present disclosure. Specifically, the method of treating a premature birth, a degenerative disease or an infectious disease, in a subject comprises,
(a) obtaining a biological sample from the subject;
(b) isolating MOIs from the biological sample by use of the present device;
(c) determining the expression level of at least one target molecule of the MOIs; and (d) treating the subject based on the expression level of the at least one target molecule determined in step (c), and
(d) administering to the subject an effective amount of a therapeutic agent when the expression level of the at least one target molecule of the MOIs or a ratio of the expressed levels of two target molecules is different *(i.e.,* higher or lower) from that of a reference sample obtained from a healthy subject.

The steps (a)-(c) of the treating method are similar to steps of the method described in Sections 1.1 or 1.2 of the present disclosure, and hence, detailed description thereof is omitted herein for the sake of brevity.

In the step (d), a skilled artisan or a clinical practitioner may administer to a subject in need thereof an effective amount of a therapeutic agent *(e.g.,* an anti-degenerative agent or an anti-infectious agent) in accordance with expression level as determined in the step (c). Specifically, as mentioned above, a subject having an expression level of one or more target molecules or a ratio of the expressed levels of two target molecules different from that of the reference sample indicates that the subject has or is at risk of developing the degenerative disease or infectious disease; accordingly, the skilled artisan or clinical practitioner may administer to such a subject a suitable treatment thereby preventing, ameliorating and/or alleviating the occurrence of or the symptoms associated with the degenerative disease or the infectious disease.

The therapeutic agent is administered to a subject having an expression pattern of one or more target molecules in MOIs (e.g., MVs) different from that of the healthy subject, in which the therapeutic agent may be an agonist or antagonist that may rescue the expression pattern of the one or more target molecules in the MOIs of the subject.

Examples of the therapeutic agent that may prevent premature birth include, but are not limited to, progesterone, corticosteroid, nifedipine, and etc.

Examples of the therapeutic agent effective in treating degenerative disease include, but are not limited to, curcumin, branched-chain amino acid (BCAA, including leucine, isoleucine, and valine), cholinesterase inhibitor (such as donepezil (Aricept), galantamine (Razadyne), and rivastigmine (Exelon)), memantine (Namenda), omega-3 fatty acid, ginkgo, vitamin (including vitamin A, vitamin C, vitamin D, and vitamin E), levodopa, carbidopa, dopamine agonist (such as pramipexole (Mirapex), ropinirole (Requip), rotigotine (Neupro), and apomorphine (Apokyn)), monoamine oxidase inhibitor (MAO inhibitor, such as selegiline (Eldepryl, Zelapar), rasagiline (Azilect), and safinamide (Xadago)), catechol O-methyltransferase inhibitor (COMT inhibitor, such as entacapone (Comtan) and tolcapone (Tasmar)), Anticholinergic (such as benztropine (Cogentin), and trihexyphenidyl), and amantadine.

Examples of the therapeutic agent effective in treating infection include, but are not limited to, anti-viral antibody, antibiotics, interferon alpha, aptamers, and etc.

The therapeutic agent may be administered to the subject by a suitable route, for example, topical, mucosal *(e.g.* intraconjunctival, intranasal, intratracheal), oral, intraspinal *(e.g.* intrathecal), intravenous, intraarterial, intramuscular, subcutaneous, intraarticular, intraventrical, intracerebroventricular, intraperitoneal, intratumoral, and intra-middle ear administration.

As would be appreciated, the method can be applied to the subject, alone or in combination with additional therapies that have some beneficial effects on the treatment of the degenerative disease, infectious disease or premature delivery. Depending on the intended purpose, the present method can be applied to the subject before, during, or after the administration of the additional therapies.

### 2.3 Use of the target molecule

Another aspect of the present disclosure, but not forming part of the claimed scope, is directed to the use of the target molecule serving as the biomarker for the manufacture of a kit. The use is characterized in that,
the biomarker is a target molecule expressed in and/or on MOIs; and
the kit is useful in making a diagnosis or prognosis of whether a subject has or is at risk of developing a premature birth, a degenerative disease or an infectious disease; wherein
in the prognosis or diagnosis of the premature birth, the target molecule is selected from the group consisting of soluble fms-like tyrosine kinase 1 (sFlt1), pregnancy associated plasma protein A (PAPPA), IL-6, IL-10, IL-1ra, and a combination thereof;
in the prognosis or diagnosis of the degenerative disease, the target molecule is selected from the group consisting of EGF-1, leptin, HB-EGF, EGF-2, VEGF-2, HGF, DPP4, CD90, EphA2, IL-2, IL-12, BDNF, B2M, IGFBP1, IGFBP2, CTGF, NFL, L1CAM, α-synuclein, fractalkine, IFN-α, IFN-γ, GRO, IL-10, MCP-3, nucleic acid (such as DNA), and a combination thereof;
in the diagnosis or prognosis of the infectious disease, the target molecule is NS-1; and
in the case when the expression level of the target molecule of the MOIs or a ratio of the expressed levels of two target molecules is different *(i.e.,* higher or lower) from that of a reference sample obtained from a healthy subject indicates that the subject has or is at risk of developing the premature birth, the degenerative disease or the infectious disease.

The following Examples are provided to elucidate certain aspects of the present invention and to aid those of skilled in the art in practicing this invention. These Examples are in no way to be considered to limit the scope of the invention in any manner. Without further elaboration, it is believed that one skilled in the art can, based on the description herein, utilize the present invention to its fullest extent.

### EXAMPLE

### Materials and Methods

### Determination of proteomic profiles in MOIs

The protein peptides of MOIs (i.e., MVs or Exo) derived from urine samples were subjected to the assays of ITRAQ^{™} (isobaric tags for relative and absolute quantification). The protein concentration in each MOIs was measured, and equalized before studies. The ITRAQ^{™} method is a protein quantitation method based on the peptides labelling with a compound that produces isobaric fragments suitable for comparison of peptides among protein samples acquired from LC/MS-MS spectrometry. Employing Applied Biosystems ITRAQ^{™} Reagents (Applied Biosystems Inc., USA) provided as a set of four isobaric reagents: ITRAQ ^{™} Reagent 114, ITRAQ ^{™} Reagent 115, ITRAQ ^{™} Reagent 116, and ITRAQ ^{™} Reagent 117 to label the proteins of each MOIs, four samples were made, so that 4-plex samples analysis was performed in an LC/MS-MS experiment. Each sample was digested by trypsin into peptides, labeled with standardized individual isobaric fragment, and readout by LC/MS-MS analyses.

### Determination of E-cadherin level

The level of E-cadherin in each sample (e.g., cells, MVs, Exos and Solns) was determined by mixing with biotin-labeled aptamer (100 uM) in one ml Tris buffer solution (TBS) for 2 hours. After washing in 5 ml TBS-tween 20 (0.1%) for 3 times, the sample was probed with streptavidin-HRP (horseradish peroxidase) solution for 2 hours. After washing for 3 times in 5 ml TBS-tween 20 0.1%, the sample was subjected to HRP substrate reagent and read by the myECL Imager equipped with chemiluminescence exposure screen.

### Determination of neutrophil gelatinase-associated lipocalin (NGAL) level

The level of NGAL in each sample (e.g., cells, MVs, Exos and Solns) was determined by biotin-labeled specific antibody (1:500) in one ml Tris buffer solution (TBS) for 2 hours, followed by washing in 5 ml TBS-tween 20 for 3 times, the sample was then probed with streptavidin-HRP (horseradish peroxidase) solution for 2 hours. After washing for 3 times in 5 ml TBS-tween 20 0.1%, the sample was subjected to HRP substrate reagent and read by the myECL Imager equipped with chemiluminescence exposure screen.

### Western blot assay

Twenty micrograms of total protein of MOIs derived from urine sample were subject to SDS-PAGE under denature condition. After electrophoresis, the protein gels were transferred onto a nitrocellulose membrane by semi-dried transfer device. The membrane was incubated with non-fat dry milk for blocking of non-specific binding in Tris buffer (50 mM) followed by incubation with an antibody (at 1: 2,000 dilution) or an aptamer (100 nM, for example, an aptamer of SEQ ID NO: 1 or 2, which were respectively specific to E-cadherin and Mucin 5Ac). After washing with Tris buffer to remove the unbound antibody or aptamer, the expression level of specific protein was detected via streptavidin-HRP (horse-radish peroxidase) chemiluminescence reaction.

### Determination of creatinine levels in urine

The biomarker level in urine would be affected by urine concentration in different physiological conditions (e.g., fasting or postprandial conditions), accordingly, urine creatinine level was measured to correct and/or compensate changes resulted from variation in physiological condition. Total protein in each sample (e.g., cells, MVs, Exos and Solns) was measured by colorimetric detection using Pierce^{™} BCA protein assay kit (Thermo Fisher Scientific, USA) with a standard spectrophotometer or plate reader at 562 nm after lysis with radioimmunoprecipitation assay buffer (RIPA buffer, Merck KGaA, Darmstadt). Urine creatinine level was measured by colormetric assay kit of alkaline picrate solution. Urine sample was diluted (1:20 dilution, in which 10 µL of sample was diluted with 190 µL of deionized water), and 50 µl of the diluted sample was added to 100 µl of alkaline picrate solution and reacted for 30 minutes. The creatinine concentration was determined by comparison to a standard curve (0.3 mg/dL-20 mg/dL) in the optical density of 490 nm using a microplate reader (ThermoFisher Scientific, USA).

### Example 1 Construction and testing of the present device

### 1.1 Isolation of MOIs by a device comprises serially connected filters

In this example, a prototype of the present device was constructed and tested. Briefly, 2 or 3 filters, each had a pore size of 0.8 µm, 0.2 µm and/or 0.05 µm, were serially connected. The filters were independently designed to capture cells (about 0.8-1 µm in size), microvesicles (about 0.2-0.8 µm in size) and exosomes (about 0.05-0. 20 µm in size), any matters that were not retained by the filters would be would be collected in the filtrate that passed through the device.

To start the process, the biological sample was fed from the inlet of the first filter (0.8µm), and was allowed to drip through or pushed through (e.g., via use of a syringe) the entire device, matters retained on each filters, as well as the final filtrate (i.e., filtrate of the third filter (0.05 µm)) were collected. In addition, the filtrate that passed through each filters, as well as the matters that were retained on each filters were sampled for subsequent determination.

To test the device, a urine sample (about 30 mL) was allowed to pass through the device, then filtrates passing through each filters, as well as matters retained on each filters, were independently collected and subjected to analysis to determine the level of E-cadherin expressed on each population of MOIs isolated by the device. The level of E-cadherin was monitored by biotin-labeled aptamer and amplified by chemiluminescence detection as described in the "Material and Methods" section.

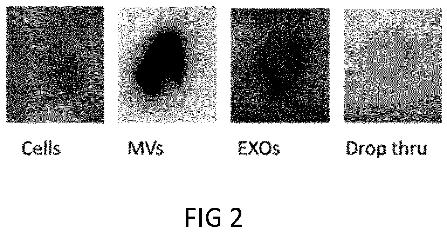

It was found that E-cadherin was differentially expressed among the cells, the microvesicles, and the exosomes, while no E-cadherin was found in the filtrate where soluble marcomolecules were collected (FIG 2).

### 1.2 Isolating urine EVs by ion chromatography

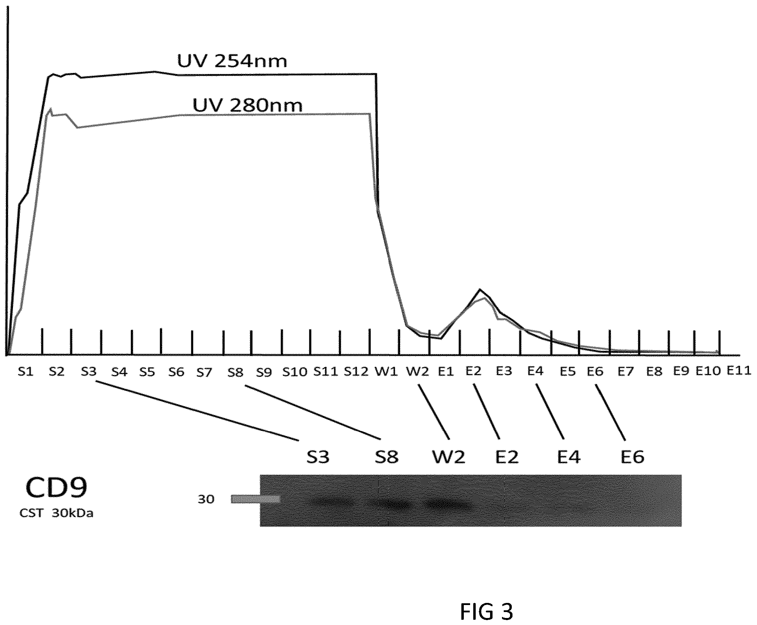

In this example, an ion exchange column was employed to isolated urine EVs. Briefly, a urine sample (about 30 mL) was allowed to pass through anion exchange column (i.e., AIEX chromatography), which was eluted with sodium chloride solution (1M), and the isolated exosomes were confirmed by monitoring the expression of CD9 thereon (See FIG 3).

### Example 2 The expression patterns of neural specific biomarkers and proteomic display among MOIs in young and aged subjects

MOIs were isolated from urine samples collected from young (< 30 years old) and aged subjects (> 6o years old) in accordance with procedures described in Example 1.1., except the expression of neutrophil gelatinase-associated lipocalin (NGAL), neuron-specific enolase (NSE) and neural cell adhesion molecule L1 (LICAM) among MOIs was monitored by biotin-labeled specific antibody and amplified by chemiluminescence detection as described in the "Material and Methods" section.

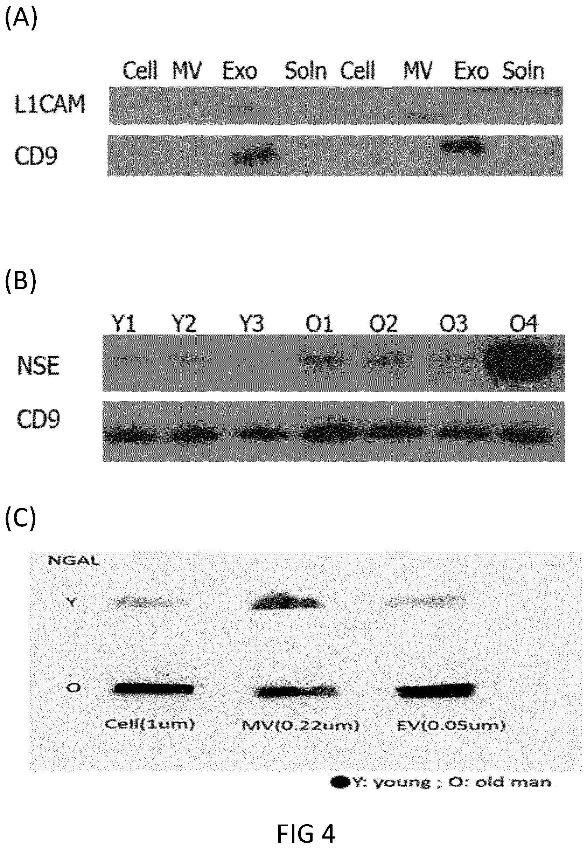

It was found that LICAM was present in Exo and MVs, and high level of NSE was found in aged adults rather than in the young ones. Further, in young subj ects, the level of NGAL was higher in the MVs, whereas in aged subject, the expression of NGAL appeared to be prominent and similar among the cells, the MVs, and the exosomes (FIG 4).

For proteomic display in MVs and Exo between young and aged subjects, a total of 1902 proteins were identified by iTRAQ (isobaric tag for relative and absolute quantitation) mass spectrometry, and 62 were significantly different between exosomes derived from young (< 30 years of age) and old adults (> 60 years of age), the data is summarized in Table 1.

**Table 1. Differential display of proteins in MVs and Exo between young and aged subjects**

| Name of Protein | p-value (Old vs. Young) | Old/Young Ratio |
|---|---|---|
| ARSF | 0.000239883 | 0.735677 |
| FGFBP2 | 0.00247768 | 2.11769 |
| PVRL3 | 0.00639341 | 1.35988 |
| FCN2 | 0.00685778 | 1.48293 |
| EMCN | 0.00724594 | 1.70521 |
| PTPRZ1 | 0.00746451 | 1.48385 |
| DSG2 | 0.00755046 | 0.651528 |
| YWHAE | 0.00813376 | 1.26886 |
| SCGB1D2 | 0.00968033 | 0.478197 |
| VDAC1 | 0.0100991 | 0.679966 |
| FAM151A | 0.012274 | 0.792115 |
| ITIH3 | 0.0123853 | 1.37189 |
| SELENBP1 | 0.0130328 | 0.677852 |
| FREM1 | 0.0161292 | 1.28571 |
| PSMA2 | 0.0161773 | 1.60143 |
| COL7A1 | 0.0161803 | 0.672241 |
| SNF8 | 0.0167085 | 1.28441 |
| CDH13 | 0.0169948 | 0.771821 |
| MAG | 0.0176917 | 1.51635 |
| LAMA4 | 0.0182737 | 1.52048 |
| CLMP | 0.0198873 | 0.712329 |
| WDR77 | 0.0205814 | 1.44587 |
| PTPRO | 0.0205913 | 1.25225 |
| IL1RAP | 0.02164 | 0.76245 |
| CHGA | 0.0226503 | 1.3563 |
| PRSS3 | 0.0230661 | 1.5641 |
| CRTAC1 | 0.0233089 | 1.772 |
| CPM | 0.0233693 | 0.578532 |
| EDIL3 | 0.0238858 | 1.53807 |
| NSFL1C | 0.0246708 | 1.2779 |
| PROS1 | 0.0251213 | 0.683502 |
| KIAA0196 | 0.0276384 | 1.5974 |
| UPK3A | 0.0318446 | 0.726057 |
| PSMC2 | 0.0319455 | 1.25606 |
| TMED7 | 0.0321121 | 0.770695 |
| GNPDA1 | 0.0322456 | 1.25663 |
| YWHAZ | 0.0328348 | 1.2555 |
| ALDOA | 0.0329756 | 1.62467 |
| PSAT1 | 0.0342038 | 1.30415 |
| GPR98 | 0.0344457 | 1.77008 |
| PCDHB3 | 0.0353558 | 1.55591 |
| SCGB2A2 | 0.0359176 | 0.659063 |
| SEMA3C | 0.0364782 | 2.02877 |
| SULT1A1 | 0.0365495 | 1.3753 |
| TMUB1 | 0.0370646 | 1.28571 |
| NAPRT | 0.0371258 | 1.43013 |
| S100A13 | 0.037762 | 1.30871 |
| APEH | 0.0385329 | 1.58398 |
| PSMA7 | 0.0390789 | 1.32693 |
| CTTN | 0.0394495 | 1.41606 |
| SLC39A14 | 0.0422883 | 1.44648 |
| VDAC3 | 0.0422917 | 0.54202 |
| C6 | 0.0431936 | 0.633987 |
| ADAM22 | 0.043205 | 0.707213 |
| PDE8A | 0.0434076 | 1.51572 |
| SDC2 | 0.0448751 | 1.61952 |
| ARSK | 0.0452458 | 1.56675 |
| NT5C | 0.0462908 | 0.742919 |
| CPXM1 | 0.0478297 | 1.33372 |
| CPB2 | 0.0480321 | 0.79176 |
| UPK2 | 0.04834 | 0.690194 |
| FGG | 0.0488512 | 0.620746 |
| CALM1 | 0.0492979 | 1.35769 |

### Example 3 Differential expression of vascular mediators and cytokines among MOIs in pregnant women having a normal or a premature birth

In order to identify factors that may induce pre-mature delivery of babies in pregnant women, the levels of various vascular mediators including soluble fms-like tyrosine kinase 1 (sFlt1), pregnancy associated plasma protein A (PAPPA), brain-derived neurotrophic factor (BNDF), and insulin-like growth factor binding protein 1 (IGFBP1), as well as cytokines including IL-6, IL-8, IL-10, , IL-8, IL-10, C-X-C Motif Chemokine Ligand 13 (CXCL 13), and interleukin-1 receptor antagonist (IL1-ra) were respectively determined among MOIs in urine samples collected from pregnant women.

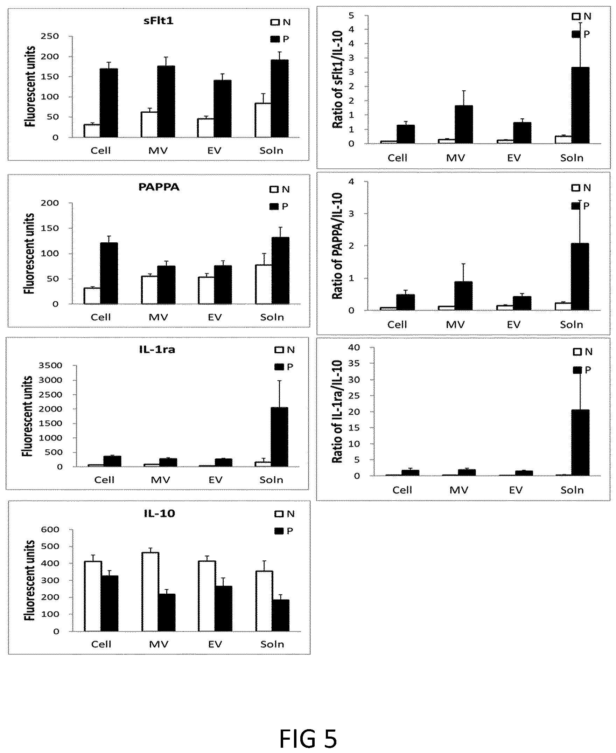

A total of 150 pregnant women were recruited for this study under prior written consent. Urine samples (each was 40 mL) were collected between 12 and 16 weeks of gestational age (GA), and 10 out of 150 pregnant women had premature delivery in the average of GA of 32 weeks and 5 days (from GA 22 weeks and 5 days to 35 weeks and 5 days). This 10 urine samples (i.e., women with premature delivery) were matched with other 10 urine samples collected from women with normal delivery during the same period of delivery. All urine samples (40 mL each) were subjected to separation of MOIs (i.e., the cells, MV, Exos (EV) and Soln) as described in Example 1.1.1. Each MOIs was then subjected to microfluidic chips assay for the determination of the respective levels of growth factors (i.e., sFlt1, PAPPA, BDNF, IGFBP1) and cytokines (IL-6, IL-8, IL-10, CXCL13, IL1ra). Results are depicted in FIG 5.

It was found that cells, MV, Exo and/or Soln isolated from urine samples of pregnant women with premature delivery (P) had higher levels of sFlt1, PAPPA and IL-1ra, and lower levels of IL-10, as compared with those from women with normal delivery (N). The sFlt1 levels in all MOIs were significantly higher in the group of premature delivery, and the IL-10 levels in MV or Exo fractions were significantly lower in the group of premature delivery (See left panels of FIG 5).

Further, ratios between a growth factor (i.e., sFlt1 and PAPPA) and a cytokine (i.e., IL-10), including sFlt1/IL-10, PAPPA/IL-10 and IL-1ra/IL-10, were calculated and depicted in the right panels in FIG 5. It was found that a higher ratio of sFlt1/EL-10, PAPPA/IL-10 or IL-1ra/IL-10 indicated that a woman has a higher risk of premature delivery.

### Example 4 Differential expression of vascular mediators and/or DNA among MOIs in young and aged subjects or subjects with Parkinson disease (PD)

### 4.1 Differential expression of growth factors among MOIs between young and aged subjects

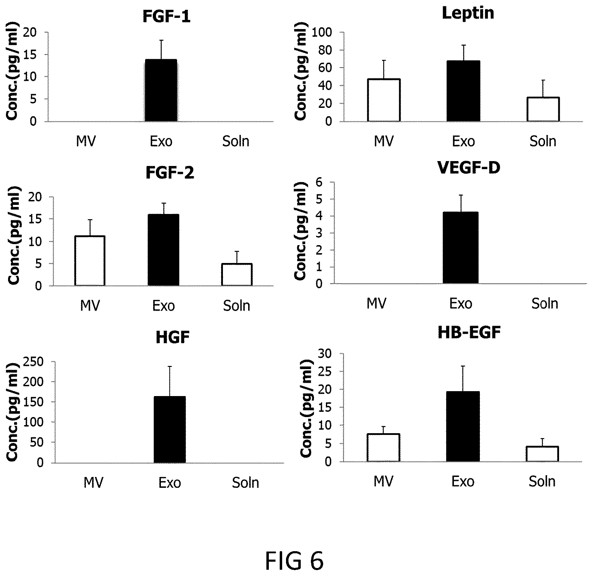

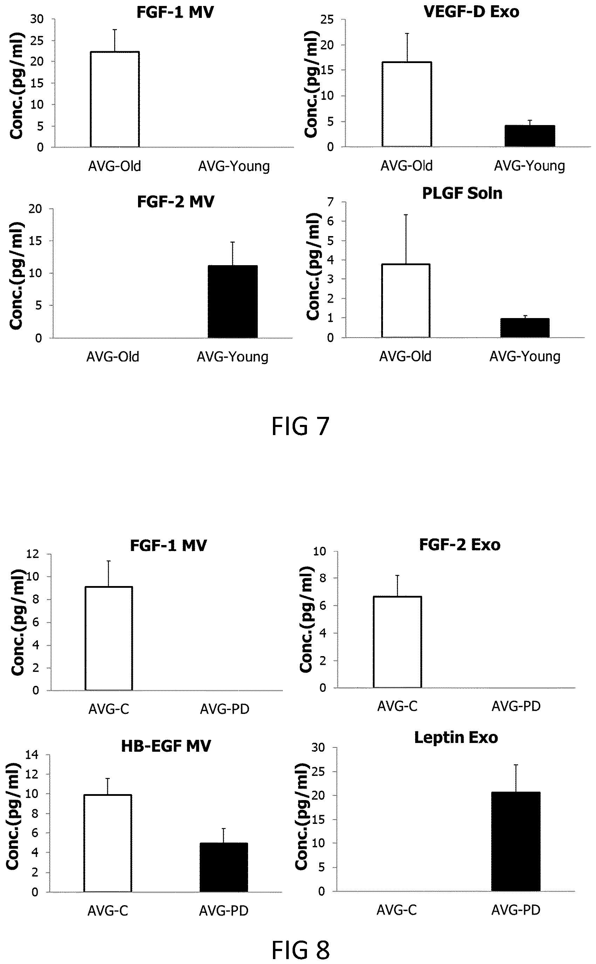

In this example, MOIs were isolated from urine samples collected from young adult subjects (i.e., 30 years old|) or aged subjects (i.e., over 60 years old), and the expression of fibroblast growth factor 1 (FGF-1), FGF-2, leptin, heparin-binding EGF-like growth factor (HB-EGF), vascular endothelial growth factor D (VEGF-D) and hepatocyte growth factor (HGF) as well as DNA levels were independently determined. Results are illustrated in FIGs 6 and 7.

First of all, it was found that the expression of FGF-1, VEGF-D and HGF were exclusively found in Exos, but not in MVs or fraction that contained soluble macromolecules; while the expressions of leptin, FGF-2 and HB-EGF were found in both MVs and Exos.

Further analysis revealed that aged subjects (i.e., AVG-old) had significant expression of FGF-1 (in MVs) and PLGF (in the soluble macromolecule fraction), as compared with those in normal subjects (i.e., AVG-Young); whereas higher expression of FGF-2 (in MVs) was in the young subjects.

### 4.2 Differential expression of growth factors among MOIs between aged subjects with or without Parkinson disease (PD)

In this example, MOIs were isolated from urine samples of aged subjects with or without PD, and the expressions of FGF-1, HB-EGF and leptin were respectively determined. Results are illustrated in FIG 8.

It was found that FGF-1 in MVs and FGF-2 in Exos) were only expressed in normal aged subjects (i.e., AVG-C), while leptin was exclusively expressed in PD subjects (i.e. AVG-PD). Further, normal aged subjects had relatively higher level of HB-EGF (in MVs) as compared with that of PD subjects.

### 4.3 The expression of leucine rich alpha-2-glycoprotein 1 (LRG1) and CD9 in young, aged and PD subjects

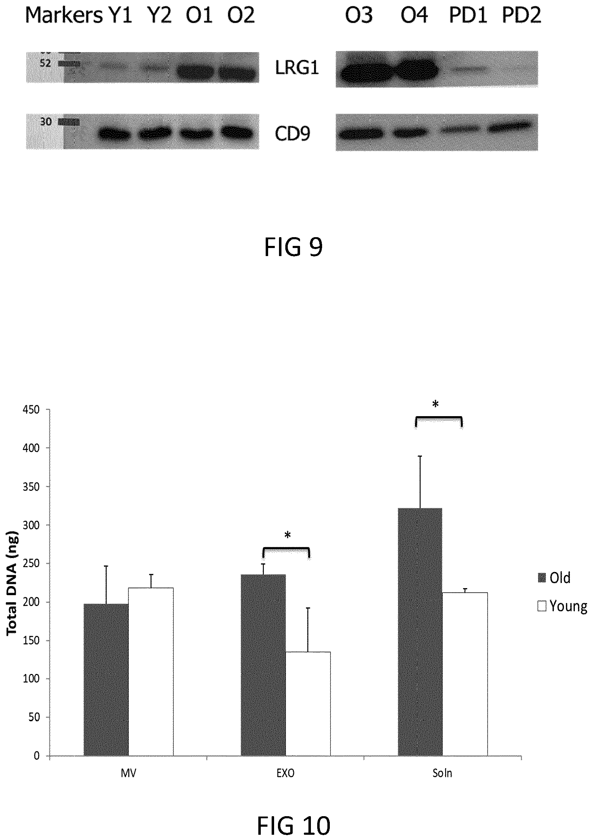

Exosomes were isolated from urine samples of young, aged and PD subjects, and the levels of LRG1 and CD9 were respectively determined via western blot analysis. Results are depicted in FIG 9.

The data indicated that young (Y) and old (O) adults had a similar expression level of exosomal CD9, but old adults (over 60 years old) had a prominent higher level of LRG1 expression. PD subjects (i.e., case 1 (PD1), case 2 (PD2) had a similar expression level of CD9, while the level of LRG1 was lower than those of normal aged adults (O1, O2, O3, and O4).

### 4.4 Differential DNA levels among MOIs in young and aged subjects

MOIs were isolated from urine samples of young and aged adult subjects, as well as from PD subjects in accordance with procedures of Example 1.1. Total DNA level and the level of 8-hydroxy-2'-deoxyguanosine (8OHdG), which is a marker for DNA damage, in each MOIs were determined. Total DNA level was determined by Nanodrop spectrometry. The level of 8OHdG was determined by enzyme-linked immunoassay in accordance with the protocol provided by the manufacturer (ThermoFisher Scientific, USA).

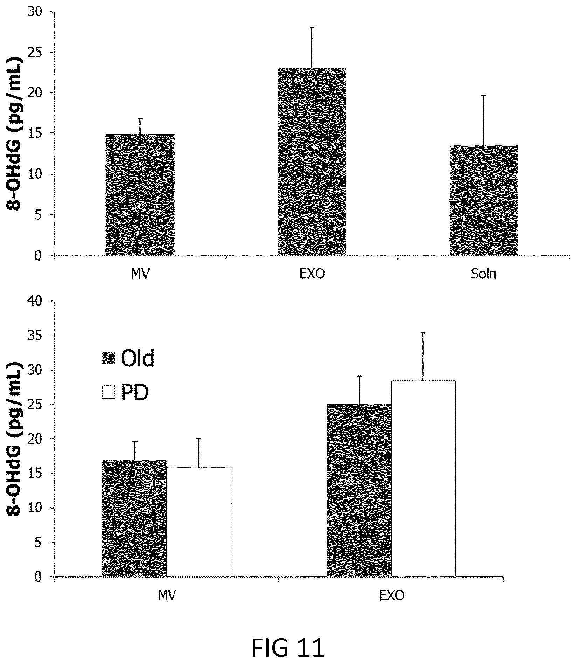

It was found that aged subjects had relatively higher total level of DNA in both exosomes and the soluble fraction (i.e., the fraction that contained soluble macromolecules) (FIG 10). Further, Exo derived from normal aged subjects had a significantly higher 8OHdG level than the MV, but the 8OHdG levels in MV or Exo were not significant different between normal aged subjects and PD subjects (FIG 11).

### Example 5 Differential expression of NS1 antigen, vascular mediators and 8OHdG among MOIs of subjects with dengue infection

MOIs were isolated from urine samples of subjects having dengue infection with or without clinical warning sign (e.g., unstable vital sign, hemo concentration, thromcytopenia and etc.) in accordance with procedures of Example 1.1. The levels of dengue virus NS1 antigen, various vascular mediators and **8OHdG** were determined. Results are illustrated in FIG 12.

Significant level of NS1 antigen was found in the MVs and some extent in Exo portion isolated from dengue subjects (FIG 11, panel (A)). As to the expression of vascular mediators, urine MVs and Exos of dengue subject had a significant higher level of EGF, while only urine exosomes had expression of G-CSF (FIG 11, panel (B)). Further, dengue subjects exhibiting clinical warning sign (i.e., AVG-W) had higher levels of leptin and VEGF-A but lower level of G-CSF, as compared with those of dengue subject without warning sign (i.e., AVG-C) (FIG 11, Panel (C)). In addition, higher level of **8OHdG** was also found in MVs of dengue subjects, as compared with that of normal subj ects.

In conclusion, the present description demonstrated that various biomarkers are differentially expressed on the cells, microvesicles, exosomes and soluble fractions in subjects suffering from a pathological condition (e.g., aging, viral infection, degenerative disease, premature delivery of a baby, and etc). The cells, microvesicles, exosomes and/or soluble factors may be easily isolated by use of the device and/or methods of the present disclosure, allowing easy identification of such subject, so that proper medication and/or measure may be administered to such subject to prevent and/or alleviate symptoms associated with the disease condition in the subj ect.

It will be understood that the above description of embodiments is given by way of example only and that various modifications may be made by those with ordinary skill in the art. The above specification, examples and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the scope of this invention.

## Claims

1. A device for isolating cells, microvesicles (MVs) and exosomes from a biological sample, the device (100) comprising a cartridge (110), which comprises a first filter (120), a second filter (130) and a third filter (140) that are serially connected to each other and independently have a plurality of pores that are about 0.8-1 µm, 0.20-0.22 µm, and 0.03-0.05 µm in diameter, respectively, and the three filters (120, 130, 140) being arranged in the order of decreased pore size so as to retain cells, MVs and exosomes independently having a size between 1.0-20 µm, 0.20-1.0 µm, or 0.05-0. 20 µm in diameter.

2. The device of claim 1, wherein each of the first, second and third filters (120, 130, 140) is a porous membrane.

3. The device of claim 1, further comprising,
a first reservoir (160) disposed upstream to the cartridge (110) for housing the biological sample; and
a second reservoir (170) disposed downstream to the cartridge (100) for collecting a filtrate comprising soluble molecules that are smaller than 0.05 µm in diameter.

4. The device of claim 3, wherein the isolated cells, MVs, exosomes and soluble molecules independently comprise DNA, RNA, protein, glycan, lipid or a combination thereof.

5. The device of claim 2, wherein
each of the first, second and third filters (120, 130, 140) is made of ceramic, resin, metal, polymer, hollow fiber, or a combination thereof;
the ceramic is made of a material selected from the group consisting of zeolite, silica, silicon carbide, alumina, aluminum titanate, spinel, mullite, zirconium phosphate, perovskite, and a combination thereof;
the resin is made of a material selected from the group consisting of an organic compound, a synthetic compound and a combination thereof;
the metal is selected from the group consisting of stainless steel, nickel, aluminum, silver, gold, cadmium, cobalt, iron, molybdenum, niobium, copper, palladium, platinum, rhodium, ruthenium, tantalum, titanium, tungsten, zirconium, an alloy, and a combination thereof;
the polymer is selected from the group consisting of, polycellulose, polyester, polyether, polypropylene, polyamide, polyimide, polyurethane, polytetrafluoroethylene, polyolefin, polyuria, polyester amide, polyethylene terephthalate, polytetrafluoroethylene, polysiloxane, polysulfone, polyester urethane, polycarbonate, polyvinyl chloride, and a combination thereof; and
the hollow fiber is a carbon fiber, a glass fiber, a metal fiber, or a combination thereof.

6. A method of isolating cells, microvesicles (MVs) and exosomes from a urine sample by use of the device (100) of claim 1, comprising,
(a) allowing the urine sample to pass through the cartridge of the device (100) of claim 1, so as to retain the cells, the MVs and the exosomes independently having a size between 1.0-20 µm, 0.20-1.0 µm, or 0.05-0. 20 µm in diameter in the first, second and third filter (120, 130, 140);
(b) collecting the filtrate eluted from the cartridge (110), wherein the filtrate comprises soluble molecules that are smaller than 0.05 µm; and
(c) respectively harvesting the cells, the MVs and the exosomes from the respective filter (120, 130, 140), and the soluble molecules from the filtrate collected in the step (b).

7. The method of claim 6, wherein each of the three filters (120, 130, 140) is a porous membrane.

8. The method of claim 7, wherein the harvested cells, MVs and exosomes from step (c), the filtrate comprising soluble macromolecules from step (b) or the filter with the cells, the MVs and the exosomes retained therein of step (a) is/are subjected to immunoblotting, chips analysis, fluoroprobing, enzymatic or electrochemical reaction for detecting at least one biomarker thereon or therein.

9. The method of claim 8, wherein the biomarker is any one of the soluble fms-like tyrosine kinase 1 (sFlt1), pregnancy associated plasma protein A (PAPPA), brain-derived neurotrophic factor (BNDF), and insulin-like growth factor binding protein 1 (IGFBP1), IGFBP2, interleukin-1 (IL-1), IL-2, IL-4, IL-6, IL-8, IL-10, IL-12, interleukin-1 receptor antagonist (IL1-ra), C-X-C Motif Chemokine Ligand 13 (CXCL 13), insulin-like growth factor (IGF), insulin-like growth factor receptor 1 (IGFR1), fibroblast growth factor 1 (FGF-1), FGF-2, leptin, heparin-binding EGF-like growth factor (HB-EGF), vascular endothelial growth factor A (VEGF-A), VEGF-C, VEGF-D, hepatocyte growth factor (HGF), E-cadherin, leucine-rich alpha-2-glycoprotein 1 (LRG1), neutrophil gelatinase-associated lipocalin (NGAL), 8-oxohydroxy deoxyguanosine (8-OHdG), granulocyte colony-stimulating factor (G-CSF), α-synuclein, L1 cell adhesion molecule (L1CAM), S100 calcium-binding protein A8 (S100A8), β4 integrin, mucin 5AC (Muc5AC), amphiregulin (AREG), cell adhesion molecule 1 (CADM1), cochlin, arginase-1 (Arg-1), β-galactosidase, interferon gamma-induced protein 10 (IP-10), monocyte chemoattractant protein 1 (MCP-1), growth-regulated alpha protein (GRO-a), syndecan 1, syndecan 4, monocyte-chemotactic protein-3 (MCP-3), tumor necrosis factor-alpha (TNFα), granulocyte-macrophage colony-stimulating factor (GM-CSF), macrophage colony-stimulating factor (M-CSF), zonulin, neurofilament light (NFL), high mobility group box 1 (HMGB 1), or a nucleic acid.

10. A method of making a diagnosis or prognosis of a disease from a urine sample of a subject, wherein the urine sample comprises cells, microvesicles (MVs), exosomes, and soluble macromolecules, and each of the cells, microvesicles (MVs), exosomes, and soluble macromolecules has a target molecule expressed thereon and/or therein, wherein the method comprises:
(a) allowing the urine sample to pass through the cartridge of the device of claim 1, having a first, a second and a third filter (120, 130, 140) independently having a plurality of pores that are about 0.8-1 µm, 0.20-0.22 µm, and 0.03-0.05 µm in diameter and being arranged in the order of decreased pore size thereby retaining the cells, the MVs and the exosomes in the first, second, and third filter (120, 130, 140), respectively;
(b) collecting the filtrate of the third filter (140), which comprises soluble macromolecules;
(c) adding capture molecules to the filtrate comprising soluble macromolecules from step (b), and to the cells, the microvesicles and the exosomes respectively retained on the three filters (120, 130, 140) in the step (a), wherein each capture molecule is linked with a reporter molecule and exhibits a binding affinity to the target molecule;
(d) determining the level of the reporter molecule bound to the target molecule in the step (c);
(e) making the diagnosis or prognosis based on the determined level of the reporter molecule in the step (d), wherein, when the determined level of the reporter molecule is different from that of a reference sample obtained from a healthy subject, then the subject has the disease or is at risk of developing the disease

11. The method of claim 10, wherein the reporter molecule is selected from the group consisting of, a tag molecule, a radioactive molecule, a fluorescent molecule, a phosphorescent molecule, a chemiluminescent molecule, and an enzyme.

12. The method of claim 10, wherein in the step (d), the level of the reporter molecule is determined by electrochemical analysis, polymerase chain reaction (PCR), real-time polymerase chain reaction (RT-PCR), flow cytometry assay, enzyme-linked immunosorbent assay (ELISA), chips array, beads array, western blotting, or kinase assay.

13. The method of claim 10, wherein the capture molecule is an aptamer, a glycan, a lectin, an antibody, or a combination thereof.

14. The method of claim 10, wherein the target molecule is soluble fms-like tyrosine kinase 1 (sFlt1), pregnancy associated plasma protein A (PAPPA), brain-derived neurotrophic factor (BNDF), and insulin-like growth factor binding protein 1 (IGFBP1), interleukin-1 (IL-1), IL-2, IL-4, IL-6, IL-8, IL-10, IL-12, interleukin-1 receptor antagonist (IL1-ra), C-X-C Motif Chemokine Ligand 13 (CXCL 13), insulin-like growth factor (IGF), insulin-like growth factor receptor 1 (IGFR1), fibroblast growth factor 1 (FGF-1), FGF-2, leptin, heparin-binding EGF-like growth factor (HB-EGF), vascular endothelial growth factor A (VEGF-A), VEGF-C, VEGF-D, hepatocyte growth factor (HGF), E-cadherin, leucine-rich alpha-2-glycoprotein (LRG), neutrophil gelatinase-associated lipocalin (NGAL), 8-oxohydroxy deoxyguanosine (8-OHdG), granulocyte colony-stimulating factor (G-CSF), α-synuclein, L1 cell adhesion molecule (L1CAM), S100 calcium-binding protein A8 (S100A8), β4 integrin, mucin 5AC (Muc5AC), amphiregulin (AREG), cell adhesion molecule 1 (CADM1), cochlin, arginase-1 (Arg-1), β-galactosidase, interferon gamma-induced protein 10 (IP-10), monocyte chemoattractant protein 1 (MCP-1), growth-regulated alpha protein (GRO-a), syndecan 1, syndecan 4, monocyte-chemotactic protein-3 (MCP-3), tumor necrosis factor-alpha (TNFα), granulocyte-macrophage colony-stimulating factor (GM-CSF), macrophage colony-stimulating factor (M-CSF), zonulin, neurofilament light (NFL), high mobility group box 1 (HMGB1), or a nucleic acid.

15. The method of claim 10, wherein the disease is a cancer, an inflammatory disease, a degenerative disease, an infectious disease, or a reproductive disease.

16. The method of claim 13, wherein
the cancer is gastric cancer, lung cancer, bladder cancer, breast cancer, pancreatic cancer, renal cancer, colorectal cancer, cervical cancer, ovarian cancer, brain tumor, prostate cancer, hepatocellular carcinoma, melanoma, esophageal carcinoma, multiple myeloma, or head and neck carcinoma;
the inflammatory disease is psoriasis, colitis, burn injury, acute kidney injury, traumatic brain injury, skin injury, arthritis or autoimmune diseases;
the degenerative disease is Parkinson's disease, Alzheimer's disease, dementia, stroke, chronic kidney disease, chronic lung disease, benign prostate hypertrophy or hearing loss;
the infectious disease is caused by bacteria, viruses or fungi; and
the reproductive disease is spontaneous abortion, intrauterine retardation, intrauterine infection, congenital anomaly, premature birth, pre-eclampsia, or pregnant diabetes.

## Patentansprüche

1. Vorrichtung zur Isolierung von Zellen, Mikrovesikeln (MVs) und Exosomen aus einer biologischen Probe, wobei die Vorrichtung (100) eine Kartusche (110) umfasst, welche einen ersten Filter (120), einen zweiten Filter (130) und einen dritten Filter (140) umfasst, die in Reihe miteinander verbunden sind und unabhängig voneinander eine Vielzahl von Poren mit einem Durchmesser von jeweils etwa 0,8-1 µm, 0,20-0,22 µm und 0,03-0,05 µm im Durchmesser aufweisen, wobei die drei Filter (120, 130, 140) in der Reihenfolge der abnehmenden Porengröße angeordnet sind, um Zellen, MVs und Exosomen unabhängig voneinander mit einer Größe zwischen 1,0-20 µm, 0,20-1,0 µm oder 0,05-0,20 µm im Durchmesser zurückzuhalten.

2. Vorrichtung nach Anspruch 1, bei welcher jeder der ersten, zweiten und dritten Filter (120, 130, 140) eine poröse Membran ist.

3. Vorrichtung nach Anspruch 1, ferner umfassend,
ein erstes Reservoir (160), das stromaufwärts der Kartusche (110) angeordnet ist, um die biologische Probe aufzunehmen; und
ein zweites Reservoir (170), das stromabwärts der Kartusche (100) angeordnet ist, um ein Filtrat zu sammeln, das lösliche Moleküle mit einem Durchmesser von weniger als 0,05 µm umfasst.

4. Vorrichtung nach Anspruch 3, bei welcher die isolierten Zellen, MVs, Exosomen und löslichen Moleküle unabhängig voneinander DNA, RNA, Protein, Glykän, Lipid oder eine Kombination davon umfassen.

5. Vorrichtung nach Anspruch 2, bei welcher
jeder der ersten, zweiten und dritten Filter (120, 130, 140) aus Keramik, Harz, Metall, Polymer, Hohlfaser oder einer Kombination davon hergestellt ist,
wobei die Keramik aus einem Material hergestellt ist, das aus der Gruppe ausgewählt ist, die aus Zeolith, Siliziumdioxid, Siliziumkarbid, Aluminiumoxid, Aluminiumtitanat, Spinell, Mullit, Zirkoniumphosphat, Perowskit und einer Kombination davon besteht,
wobei das Harz aus einem Material hergestellt ist, das aus der Gruppe ausgewählt ist, die aus einer organischen Verbindung, einer synthetischen Verbindung und einer Kombination davon besteht,
wobei das Metall aus der Gruppe ausgewählt ist, die aus rostfreiem Stahl, Nickel, Aluminium, Silber, Gold, Cadmium, Kobalt, Eisen, Molybdän, Niob, Kupfer, Palladium, Platin, Rhodium, Ruthenium, Tantal, Titan, Wolfram, Zirkonium, einer Legierung und einer Kombination davon besteht;
wobei das Polymer aus der Gruppe ausgewählt ist, die aus Polycellulose, Polyester, Polyether, Polypropylen, Polyamid, Polyimid, Polyurethan, Polytetrafluorethylen, Polyolefin, Polyurid, Polyesteramid, Polyethylenterephthalat, Polytetrafluorethylen, Polysiloxan, Polysulfon, Polyesterurethan, Polycarbonat, Polyvinylchlorid und einer Kombination davon besteht, und
wobei die Hohlfaser eine Kohlenstofffaser, eine Glasfaser, eine Metallfaser oder eine Kombination davon ist.

6. Verfahren zur Isolierung von Zellen, Mikrovesikeln (MVs) und Exosomen aus einer Urinprobe unter Verwendung der Vorrichtung (100) nach Anspruch 1, umfassend,
(a) Durchlaufen der Urinprobe durch die Kartusche der Vorrichtung (100) nach Anspruch 1, um die Zellen, die MVs und die Exosomen, die unabhängig voneinander eine Größe zwischen 1,0-20 µm, 0,20-1,0 µm oder 0,05-0,20 µm im Durchmesser aufweisen, in dem ersten, zweiten und dritten Filter (120, 130, 140) zurückzuhalten,
(b) Sammeln des aus der Kartusche (110) eluierten Filtrats, wobei das Filtrat lösliche Moleküle umfasst, die kleiner als 0,05 µm sind, und
(c) jeweiliges Entnehmen der Zellen, der MVs und der Exosomen von dem jeweiligen Filter (120, 130, 140) und der löslichen Moleküle aus dem in Schritt (b) gesammelten Filtrat.

7. Verfahren nach Anspruch 6, bei welchem jeder der drei Filter (120, 130, 140) eine poröse Membran ist.

8. Verfahren nach Anspruch 7, bei welchem die gesammelten Zellen, MVs und Exosomen aus Schritt (c), das lösliche Makromoleküle enthaltende Filtrat aus Schritt (b) oder der Filter mit den darin zurückgehaltenen Zellen, MVs und Exosomen aus Schritt (a) einem Immunoblotting, einer Chipanalyse, einer Fluor-Sondierung, einer enzymatischen oder elektrochemischen Reaktion zum Nachweis mindestens eines Biomarkers darauf oder darin unterzogen wird/werden.

9. Verfahren nach Anspruch 8, bei welchem der Biomarker einer der Folgenden ist, lösliche Fms-ähnliche Tyrosinkinase 1 (sFlt1), schwangerschaftsassoziiertes Plasmaprotein A (PAPPA), vom Gehirn abgeleiteter neurotropher Faktor (BNDF), und insulinähnliches Wachstumsfaktor-Bindungsprotein 1 (IGFBP1), IGFBP2, Interleukin-1 (IL-1), IL-2, IL-4, IL-6, IL-8, IL-10, IL-12, Interleukin-1-Rezeptorantagonist (IL1-ra), C-X-C-Motiv-Chemokin Ligand 13 (CXCL 13), insulinähnlicher Wachstumsfaktor (IGF), insulinähnlicher Wachstumsfaktor-Rezeptor 1 (IGFR1), Fibroblasten-Wachstumsfaktor 1 (FGF-1), FGF-2, Leptin, heparin-bindender EGF-ähnlicher Wachstumsfaktor (HB-EGF), vaskulärer endothelialer Wachstumsfaktor A (VEGF-A), VEGF-C, VEGF-D, Hepatozyten-Wachstumsfaktor (HGF), E-Cadherin, leucinreiches Alpha-2-Glykoprotein 1 (LRG1), Neutrophilen-Gelatinase-assoziiertes Lipocalin (NGAL), 8-Hydroxydesoxyguanosin (8-OHdG), Granulozyten-Kolonie-stimulierender Faktor (G-CSF), α-Synuclein, Zelladhäsionsmolekül L1 (L1 CAM), S100 Kalzium-bindendes Protein A8 (S100A8), ß4-Integrin, Mucin 5AC (Muc5AC), Amphiregulin (AREG), Zelladhäsionsmolekül 1 (CADM1), Cochlin, Arginase 1 (Arg-1), ß-Galactosidase, Interferon-gamma induziertes Protein 10 (IP-10), Monozyten-Chemoattraktor-Protein 1 (MCP-1), wachstumreguliertes Alpha-Protein (GRO-a), Syndecan-1, Syndecan-4, Monozyten-chemotaktisches Protein-3 (MCP-3), Tumornekrosefaktor-alpha (TNFα), Granulozyten-Makrophagen-Kolonie- stimulierender Faktor (GM-CSF), Makrophagen-Kolonie-stimulierender Faktor (M-CSF), Zonulin, Neurofilament light (NFL), High-Mobility Group Box 1 (HMGB 1), oder eine Nukleinsäure.

10. Verfahren zur Erstellung einer Diagnose oder Prognose einer Krankheit aus einer Urinprobe eines Subjekts, wobei die Urinprobe Zellen, Mikrovesikel (MVs), Exosomen und lösliche Makromoleküle umfasst und jede der Zellen, Mikrovesikel (MVs), Exosomen und löslichen Makromoleküle ein darauf und/oder darin exprimiertes Zielmolekül aufweist, wobei das Verfahren umfasst:
(a) Durchlaufen der Urinprobe durch die Kartusche der Vorrichtung nach Anspruch 1, die einen ersten, einen zweiten und einen dritten Filter (120, 130, 140) aufweist, die unabhängig voneinander eine Vielzahl von Poren mit einem Durchmesser von etwa 0,8-1 µm, 0,20-0,22 µm und 0,03-0,05 µm aufweisen und in der Reihenfolge der abnehmenden Porengröße angeordnet sind, wodurch die Zellen, die MVs und die Exosomen im ersten, zweiten und dritten Filter (120, 130, 140) jeweils zurückgehalten werden,
(b) Sammeln des Filtrats des dritten Filters (140), das lösliche Makromoleküle enthält,
(c) Zugabe von Fängermolekülen zu dem Filtrat, das lösliche Makromoleküle umfasst, aus Schritt (b) und zu den Zellen, den Mikrovesikeln und den Exosomen, die jeweils auf den drei Filtern (120, 130, 140) in Schritt (a) zurückgehalten werden, wobei jedes Fängermolekül mit einem Reportermolekül verknüpft ist und eine Bindungsaffinität zu dem Zielmolekül aufweist,
(d) Bestimmen der Menge des an das Zielmolekül gebundenen Reportermoleküls in Schritt (c),
(e) Erstellen der Diagnose oder Prognose auf der Grundlage des in Schritt (d) bestimmten Levels des Reportermoleküls, wobei, wenn der bestimmte Level des Reportermoleküls sich von dem einer von einem gesunden Subjekt erhaltenen Referenzprobe unterscheidet, das Subjekt die Krankheit hat oder das Risiko hat, die Krankheit zu entwickeln.

11. Verfahren nach Anspruch 10, bei welchem das Reportermolekül ausgewählt ist aus der Gruppe bestehend aus einem Marker-Molekül, einem radioaktiven Molekül, einem fluoreszierenden Molekül, einem phosphoreszierenden Molekül, einem chemilumineszierenden Molekül und einem Enzym.

12. Verfahren nach Anspruch 10, bei welchem in Schritt (d) der Level des Reportermoleküls durch elektrochemische Analyse, Polymerase-Kettenreaktion (PCR), Echtzeit-Polymerase-Kettenreaktion (RT-PCR), Durchflusszytometrie-Assay, Enzymimmunoassay (ELISA), Chip-Array, Beads-Array, Western Blotting oder Kinase-Assay bestimmt wird.

13. Verfahren nach Anspruch 10, bei welchem das Fängermolekül ein Aptamer, ein Glykan, ein Lektin, ein Antikörper oder eine Kombination davon ist.

14. Verfahren nach Anspruch 10, bei welchem das Zielmolekül lösliche Fms-ähnliche Tyrosinkinase 1 (sFlt1), schwangerschaftsassoziiertes Plasmaprotein A (PAPPA), vom Gehirn abgeleiteter neurotropher Faktor (BNDF) und insulinähnliches Wachstumsfaktor-Bindungsprotein 1 (IGFBP1), Interleukin-1 (IL-1), IL-2, IL-4, IL-6, IL-8, IL-10, IL-12, Interleukin-1-Rezeptorantagonist (IL1-ra), C-X-C-Motiv-Chemokin-Ligand 13 (CXCL 13), insulinähnlicher Wachstumsfaktor (IGF), insulinähnlicher Wachstumsfaktor-Rezeptor 1 (IGFR1), Fibroblasten-Wachstumsfaktor 1 (FGF-1), FGF-2, Leptin, Heparin-bindender EGF-ähnlicher Wachstumsfaktor (HB-EGF), vaskulärer endothelialer Wachstumsfaktor A (VEGF-A), VEGF-C, VEGF-D, Hepatozyten-Wachstumsfaktor (HGF), E-Cadherin, leucinreiches Alpha-2-Glykoprotein (LRG), Neutrophilen-Gelatinase-assoziiertes Lipocalin (NGAL), 8-Hydroxydesoxyguanosin (8-OHdG), Granulozyten-Kolonie-stimulierender Faktor (G-CSF), α-Synuclein, Zelladhäsionsmolekül L1 (L1CAM), S100 Kalzium-bindendes Protein A8 (S100A8), ß4-Integrin, Mucin 5AC (Muc5AC), Amphiregulin (AREG), Zelladhäsionsmolekül 1 (CADM1), Cochlin, Arginase 1 (Arg-1), ß-Galactosidase, Interferon-gamma induziertes Protein 10 (IP-10), Monozyten-Chemoattraktives Protein 1 (MCP-1), wachstumsreguliertes Alpha-Protein (GRO-a), Syndecan-1, Syndecan-4, Monozyten-chemotaktisches Protein-3 (MCP-3), Tumornekrosefaktor-alpha (TNFα), Granulozyten-Makrophagen-Kolonie-stimulierender Faktor (GM-CSF), Makrophagen-Kolonie-stimulierender Faktor (M-CSF), Zonulin, Neurofilament light (NFL), High Mobility Group Box 1 (HMGB1), oder eine Nukleinsäure ist.

15. Verfahren nach Anspruch 10, bei welchem die Krankheit ein Krebs, eine Entzündungskrankheit, eine Degenerationskrankheit, eine Infektionskrankheit oder eine Fortpflanzungskrankheit ist.

16. Verfahren nach Anspruch 13, wobei
es sich bei dem Krebs um Magenkrebs, Lungenkrebs, Blasenkrebs, Brustkrebs, Bauchspeicheldrüsenkrebs, Nierenkrebs, Darmkrebs, Gebärmutterhalskrebs, Eierstockkrebs, Hirntumor, Prostatakrebs, Leberzellkarzinom, Melanom, Speiseröhrenkarzinom, multiples Myelom oder Kopf-Hals-Karzinom handelt,
es sich bei der Entzündungskrankheit um Schuppenflechte, Colitis, Verbrennungsverletzungen, akute Nierenverletzungen, traumatische Hirnverletzungen, Hautverletzungen, Arthritis oder Autoimmunerkrankungen handelt,
die Degenerationskrankheit die Parkinson-Krankheit, die Alzheimer-Krankheit, Demenz, Schlaganfall, chronische Nierenerkrankung, chronische Lungenerkrankung, gutartige Prostatahypertrophie oder Hörverlust ist,
die Infektionskrankheit durch Bakterien, Viren oder Pilze verursacht wird und die Fortpflanzungskrankheit Spontanabort, intrauterine Retardierung, intrauterine Infektion, kongenitale Anomalie, Frühgeburt, Präeklampsie oder Schwangerschaftsdiabetes ist.

## Revendications

1. Dispositif pour isoler des cellules, des microvésicules (MV) et des exosomes à partir d'un échantillon biologique, le dispositif (100) comprenant une cartouche (110), qui comprend un premier filtre (120), un deuxième filtre (130) et un troisième filtre (140) qui sont reliés en série les uns aux autres et présentent indépendamment une pluralité de pores qui ont un diamètre d'environ 0,8-1 µm, 0,20-0,22 µm, et 0,03-0,05 µm, respectivement, et les trois filtres (120, 130, 140) étant agencés dans l'ordre de taille de pore décroissante de manière à retenir des cellules, des MV et des exosomes présentant indépendamment une taille entre 1,0-20 µm, 0,20-1,0 µm, ou 0,05-0,20 µm de diamètre.

2. Dispositif selon la revendication 1, dans lequel chacun des premier, deuxième et troisième filtres (120, 130, 140) est une membrane poreuse.

3. Dispositif selon la revendication 1, comprenant en outre
un premier réservoir (160) disposé en amont de la cartouche (110) pour loger l'échantillon biologique; et
un second réservoir (170) disposé en aval de la cartouche (100) pour collecter un filtrat comprenant des molécules solubles qui ont un diamètre inférieur à 0,05 µm.

4. Dispositif selon la revendication 3, dans lequel les cellules, MV, exosomes et molécules solubles isolés comprennent indépendamment un ADN, un ARN, une protéine, un glycane, un lipide ou une combinaison de ceux-ci.

5. Dispositif selon la revendication 2, dans lequel
chacun des premier, deuxième et troisième filtres (120, 130, 140) est fait d'une céramique, d'une résine, d'un métal, d'un polymère, d'une fibre creuse, ou d'une combinaison de ceux-ci;
la céramique est faite d'un matériau sélectionné dans le groupe consistant en une zéolite, une silice, un carbure de silicium, une alumine, le titanate d'aluminium, un spinelle, une mullite, le phosphate de zirconium, une pérovskite, et une combinaison de ceux-ci;
la résine est faite d'un matériau sélectionné dans le groupe consistant en un composé organique, un composé synthétique et une combinaison de ceux-ci; le métal est sélectionné dans le groupe consistant en un acier inoxydable, le nickel, l'aluminium, l'argent, l'or, le cadmium, le cobalt, le fer, le molybdène, le niobium, le cuivre, le palladium, le platine, le rhodium, le ruthénium, le tantale, le titane, le tungstène, le zirconium, un alliage, et une combinaison de ceux-ci;
le polymère est sélectionné dans le groupe consistant en une polycellulose, un polyester, un polyéther, un polypropylène, un polyamide, un polyimide, un polyuréthane, un polytétrafluoroéthylène, une polyoléfine, une polyurée, un polyester amide, un polytéréphtalate d'éthylène, un polytétrafluoroéthylène, un polysiloxane, une polysulfone, un polyester uréthane, un polycarbonate, un polychlorure de vinyle, et une combinaison de ceux-ci; et
la fibre creuse est une fibre de carbone, une fibre de verre, une fibre métallique, ou une combinaison de celles-ci.

6. Procédé d'isolement de cellules, de microvésicules (MV) et d'exosomes à partir d'un échantillon d'urine en utilisant le dispositif (100) selon la revendication 1, comprenant les étapes consistant à,
(a) laisser passer l'échantillon d'urine à travers la cartouche du dispositif (100) selon la revendication 1, de manière à retenir les cellules, les MV et les exosomes présentant indépendamment une taille entre 1,0-20 µm, 0,20-1,0 µm, ou 0,05-0,20 µm de diamètre dans les premier, deuxième et troisième filtres (120, 130, 140);
(b) collecter le filtrat élué à partir de la cartouche (110), dans lequel le filtrat comprend des molécules solubles qui sont plus petites que 0,05 µm ; et
(c) récolter respectivement les cellules, les MV et les exosomes à partir du filtre (120, 130, 140) respectif, et les molécules solubles à partir du filtrat collecté dans l'étape (b).

7. Procédé selon la revendication 6, dans lequel chacun des trois filtres (120, 130, 140) est une membrane poreuse.

8. Procédé selon la revendication 7, dans lequel les cellules, MV et exosomes récoltés provenant de l'étape (c), le filtrat comprenant des macromolécules solubles provenant de l'étape (b) ou le filtre avec les cellules, les MV et les exosomes retenus dans celui-ci de l'étape (a) est/sont soumis à un immunobuvardage, une analyse sur puces, une fluorosonde, une réaction enzymatique ou électrochimique pour détecter au moins un biomarqueur sur ceux-ci ou dans ceux-ci.

9. Procédé selon la revendication 8, dans lequel le biomarqueur est l'un quelconque parmi la tyrosine kinase 1 de type fms soluble (sFlt1), la protéine plasmatique A associée à la grossesse (PAPPA), le facteur neurotrophique dérivé du cerveau (BNDF), et la protéine 1 de liaison au facteur de croissance insulinomimétique (IGFBP1), l'IGFBP2, l'interleukine-1 (IL-1), l'IL-2, l'IL-4, l'IL-6, l'IL-8, l'IL-10, l'IL-12, un antagoniste des récepteurs de l'interleukine-1 (IL1-ra), le ligand 13 de chimiokine à motif C-X-C (CXCL 13), le facteur de croissance insulinomimétique (IGF), le récepteur 1 du facteur de croissance insulinomimétique (IGFR1), le facteur 1 de croissance des fibroblastes (FGF-1), le FGF-2, la leptine, le facteur de croissance de type EGF se liant à l'héparine (HB-EGF), le facteur de croissance endothélial vasculaire A (VEGF-A), le VEGF-C, le VEGF-D, le facteur de croissance des hépatocytes (HGF), la cadhérine E, l'alpha-2-glycoprotéine 1 riche en leucine (LRG1), la lipocaline associée à la gélatinase des neutrophiles (NGAL), la 8-oxohydroxydésoxyguanosine (8-OHdG), le facteur de stimulation des colonies de granulocytes (G-CSF), l'a-synucléine, la molécule d'adhérence cellulaire L1 (L1CAM), la protéine A8 de liaison au calcium S100 (S100A8), l'intégrine β4, la mucine 5AC (Muc5AC), l'amphiréguline (AREG), la molécule d'adhérence cellulaire 1 (CADM1), la cochline, l'arginase-1 (Arg-1), la β-galactosidase, la protéine 10 induite par l'interféron gamma (IP-10), la protéine chimiotactique 1 des monocytes (MCP-1), la protéine alpha régulée par la croissance (GRO-a), le syndécane 1, le syndécane 4, la protéine chimiotactique 3 des monocytes (MCP-3), le facteur de nécrose tumorale alpha (TNFα), le facteur de stimulation des colonies de granulocytes-macrophages (GM-CSF), le facteur de stimulation des colonies de macrophages (M-CSF), la zonuline, une chaîne légère de neurofilament (NFL), la boîte 1 du groupe à haute mobilité (HMGB1), ou un acide nucléique.

10. Procédé de diagnostic ou de pronostic d'une maladie à partir d'un échantillon d'urine d'un sujet, dans lequel l'échantillon d'urine comprend des cellules, des microvésicules (MV), des exosomes, et des macromolécules solubles, et chacun des cellules, microvésicules, exosomes et macromolécules solubles présente une molécule cible exprimée sur celui-ci et/ou dans celui-ci, dans lequel le procédé comprend les étapes consistant à:
(a) laisser passer l'échantillon d'urine à travers la cartouche du dispositif selon la revendication 1, présentant un premier, un deuxième et un troisième filtre (120, 130, 140) présentant indépendamment une pluralité de pores qui ont un diamètre d'environ 0,8-1 µm, 0,20-0,22 µm, et 0,03-0,05 µm et étant agencés dans l'ordre de taille de pore décroissante pour ainsi retenir les cellules, les MV et les exosomes dans les premier, deuxième, et troisième filtres (120, 130, 140), respectivement;
(b) collecter le filtrat du troisième filtre (140), qui comprend des molécules solubles;
(c) ajouter des molécules de capture au filtrat comprenant des macromolécules solubles provenant de l'étape (b), et aux cellules, aux microvésicules et aux exosomes retenus respectivement sur les trois filtres (120, 130, 140) dans l'étape (a), dans lequel chaque molécule de capture est liée à une molécule rapporteur et présente une affinité de liaison avec la molécule cible;
(d) déterminer le niveau de la molécule rapporteur liée à la molécule cible dans l'étape (c);
(e) établir le diagnostic ou le pronostic sur la base du niveau déterminé de la molécule rapporteur dans l'étape (d), dans lequel, quand le niveau déterminé de la molécule rapporteur est différent de celui d'un échantillon de référence obtenu à partir d'un sujet en bonne santé, alors le sujet présente la maladie ou risque de développer la maladie.

11. Procédé selon la revendication 10, dans lequel la molécule rapporteur est sélectionnée dans le groupe consistant en une molécule de marqueur, une molécule radioactive, une molécule fluorescente, une molécule phosphorescente, une molécule chimioluminescente, et une enzyme.

12. Procédé selon la revendication 10, dans lequel dans l'étape (d), le niveau de la molécule rapporteur est déterminé par une analyse électrochimique, une réaction en chaîne par polymérase (PCR), une réaction en chaîne par polymérase en temps réel (RT-PCR), un dosage par cytométrie en flux, un dosage immunoenzymatique (ELISA), un réseau de puces, un réseau de billes, un transfert de western, ou un dosage avec kinase.

13. Procédé selon la revendication 10, dans lequel la molécule de capture est un aptamère, un glycane, une lectine, un anticorps, ou une combinaison de ceux-ci.

14. Procédé selon la revendication 10, dans lequel la molécule cible est la tyrosine kinase 1 de type fms soluble (sFlt1), la protéine plasmatique A associée à la grossesse (PAPPA), le facteur neurotrophique dérivé du cerveau (BNDF), et la protéine 1 de liaison au facteur de croissance insulinomimétique (IGFBP1), l'interleukine-1 (IL-1), l'IL-2, l'IL-4, l'IL-6, l'IL-8, l'IL-10, l'IL-12, un antagoniste des récepteurs de l'interleukine-1 (IL1-ra), le ligand 13 de chimiokine à motif C-X-C (CXCL 13), le facteur de croissance insulinomimétique (IGF), le récepteur 1 du facteur de croissance insulinomimétique (IGFR1), le facteur 1 de croissance des fibroblastes (FGF-1), le FGF-2, la leptine, le facteur de croissance de type EGF se liant à l'héparine (HB-EGF), le facteur de croissance endothélial vasculaire A (VEGF-A), le VEGF-C, le VEGF-D, le facteur de croissance des hépatocytes (HGF), la cadhérine E, l'alpha-2-glycoprotéine 1 riche en leucine (LRG), la lipocaline associée à la gélatinase des neutrophiles (NGAL), la 8-oxohydroxydésoxyguanosine (8-OHdG), le facteur de stimulation des colonies de granulocytes (G-CSF), l'a-synucléine, la molécule d'adhérence cellulaire L1 (L1 CAM), la protéine A8 de liaison au calcium S100 (S100A8), l'intégrine β4, la mucine 5AC (Muc5AC), l'amphiréguline (AREG), la molécule d'adhérence cellulaire 1 (CADM1), la cochline, l'arginase-1 (Arg-1), la β-galactosidase, la protéine 10 induite par l'interféron gamma (IP-10), la protéine chimiotactique 1 des monocytes (MCP-1), la protéine alpha régulée par la croissance (GRO-a), le syndécane 1, le syndécane 4, la protéine chimiotactique 3 des monocytes (MCP-3), le facteur de nécrose tumorale alpha (TNFα), le facteur de stimulation des colonies de granulocytes-macrophages (GM-CSF), le facteur de stimulation des colonies de macrophages (M-CSF), la zonuline, une chaîne légère de neurofilament (NFL), la boîte 1 du groupe à haute mobilité (HMGB1), ou un acide nucléique.

15. Procédé selon la revendication 10, dans lequel la maladie est un cancer, une maladie inflammatoire, une maladie dégénérative, une maladie infectieuse, ou une maladie de la reproduction.

16. Procédé selon la revendication 13,
dans lequel le cancer est un cancer gastrique, un cancer du poumon, un cancer de la vessie, un cancer du sein, un cancer du pancréas, un cancer du rein, un cancer colorectal, un cancer du col de l'utérus, un cancer de l'ovaire, une tumeur cérébrale, un cancer de la prostate, un carcinome hépatocellulaire, un mélanome, un carcinome de l'œsophage, un myélome multiple, ou un carcinome de la tête et du cou;
la maladie inflammatoire est un psoriasis, une colite, une brûlure, une lésion rénale aiguë, une lésion cérébrale traumatique, une lésion cutanée, l'arthrite ou les maladies auto-immunes;
la maladie dégénérative est la maladie de Parkinson, la maladie d'Alzheimer, une démence, un accident vasculaire cérébral, une maladie rénale chronique, une maladie pulmonaire chronique, une hypertrophie bénigne de la prostate ou une perte auditive;
la maladie infectieuse est provoquée par des bactéries, des virus ou des champignons; et
la maladie de la reproduction est un avortement spontané, un retard intra-utérin, une infection intra-utérine, une anomalie congénitale, une naissance prématurée, une pré-éclampsie, ou un diabète de grossesse.
